# EUROPÄISCHE PATENTSCHRIFT

(11) **EP 2 844 759 B1**
(45) Veröffentlichungstag und Bekanntmachung des Hinweises auf die Patenterteilung: **18.07.2018**
(21) Anmeldenummer: 12823073.7
(22) Anmeldetag: 12.12.2012
(51) Int. Cl.: C12N 15/81, A61K 39/00, A61K 39/12, A61K 39/02, G01N 33/569

(54) **VAKZINIERUNG MITTELS REKOMBINANTER HEFE DURCH ERZEUGUNG EINER PROTEKTIVEN HUMORALEN IMMUNANTWORT GEGEN DEFINIERTE ANTIGENE**
VACCINATION BY MEANS OF RECOMBINANT YEAST BY PRODUCING A PROTECTIVE HUMORAL IMMUNE RESPONSE AGAINST DEFINED ANTIGENS
VACCINATION AU MOYEN DE LEVURES RECOMBINANTES POUR INDUIRE UNE RÉPONSE IMMUNITAIRE HUMORALE PROTECTRICE CONTRE DES ANTIGÈNES DÉFINIS

(30) Priorität: 13.12.2011 DE 102011121069
(43) Veröffentlichungstag der Anmeldung: 11.03.2015
(73) Patentinhaber: Martin-Luther-Universität Halle-Wittenberg, 06108 Halle/Saale (DE)
(72) Erfinder: BREUNIG, Karin, Berlin 14109 (DE); BEHRENS, Sven-Erik, 06120 Halle/Saale (DE)
(86) Internationale Anmeldenummer: PCT/DE2012/001205
(87) Internationale Veröffentlichungsnummer: WO 2013/107436

(56) Entgegenhaltungen:
- EP-A1- 1 752 468
- WO-A1-90/15140
- WO-A2-2010/054649
- VILLEGAS PEDRO ET AL: "Infectious bursal disease subunit vaccination.", AVIAN DISEASES DEC 2008, Bd. 52, Nr. 4, Dezember 2008 (2008-12), Seiten 670-674, XP002695513, ISSN: 0005-2086
- PITCOVSKI J ET AL: "Development and large-scale use of recombinant VP2 vaccine for the prevention of infectious bursal disease of chickens", VACCINE, ELSEVIER LTD, GB, Bd. 21, Nr. 32, 1. Dezember 2003 (2003-12-01), Seiten 4736-4743, XP004469693, ISSN: 0264-410X, DOI: 10.1016/S0264-410X(03)00525-5
- STUBBS A C ET AL: "WHOLE RECOMBINANT YEAST VACCINE ACTIVATES DENDRITIC CELLS AND ELICITS PROTECTIVE CELL-MEDIATED IMMUNITY", NATURE MEDICINE, NATURE PUBLISHING GROUP, NEW YORK, NY, US, Bd. 7, Nr. 5, 1. Mai 2001 (2001-05-01), Seiten 625-629, XP008073223, ISSN: 1078-8956, DOI: 10.1038/87974 in der Anmeldung erwähnt
- STUBBS A C ET AL: "Recombinant yeast as a vaccine vector for the induction of cytotoxic T-lymphocyte responses", CURRENT OPINION IN MOLECULAR THERAPEUTICS, CURRENT DRUGS, LONDON, GB, Bd. 4, Nr. 1, 1. Februar 2002 (2002-02-01) , Seiten 35-40, XP008092368, ISSN: 1464-8431 in der Anmeldung erwähnt
- WANSLEY ELIZABETH K ET AL: "Vaccination with a recombinant Saccharomyces cerevisiae expressing a tumor antigen breaks immune tolerance and elicits therapeutic antitumor responses", CLINICAL CANCER RESEARCH, THE AMERICAN ASSOCIATION FOR CANCER RESEARCH, US, Bd. 14, Nr. 13, 1. Juli 2008 (2008-07-01), Seiten 4316-4325, XP002587490, ISSN: 1078-0432, DOI: 10.1158/1078-0432.CCR-08-0393 in der Anmeldung erwähnt
- BATHURST I C: "PROTEIN EXPRESSION IN YEAST AS AN APPROCH TO PRODUCTION OF RECOMBINANT MALARIA ANTIGENS", AMERICAN JOURNAL OF TROPICAL MEDICINE & HYGIENE, AMERICAN SOCIETY OF TROPICAL MEDICINE AND HYGIENE, US, Bd. 50, Nr. 4, SUPPL, 1. Januar 1994 (1994-01-01), Seiten 20-26, XP001088993, ISSN: 0002-9637 in der Anmeldung erwähnt
- ARNOLD MARINA ET AL: "Protective Vaccination against Infectious Bursal Disease Virus with Whole Recombinant Kluyveromyces lactis Yeast Expressing the Viral VP2 Subunit", PLOS ONE, Bd. 7, Nr. 9, September 2012 (2012-09), XP002695514, ISSN: 1932-6203
- JORRIT-JAN KRIJGER ET AL: "A novel, lactase-based selection and strain improvement strategy for recombinant protein expression in Kluyveromyces lactis", MICROBIAL CELL FACTORIES, BIOMED CENTRAL, LONDON, NL, Bd. 11, Nr. 1, 20. August 2012 (2012-08-20), Seite 112, XP021128994, ISSN: 1475-2859, DOI: 10.1186/1475-2859-11-112

## Beschreibung

### Gebiet der Erfindung

Die Erfindung betrifft rekombinante Hefen der Spezies *Kluyveromyces lactis,* die das VP2-Antigen des Virus der Infektiösen Bursitis (IBDV) enthalten, kodiert durch ein ins Hefegenom integriertes Fremdgen , zur Erzeugung einer humoralen Immunantwort, die Herstellung dieser Hefen und deren Verwendung zur protektiven Vakzinierung gegen Pathogene, die diese Antigene enthalten.

### Stand der Technik

Vakzine werden eingesetzt, um Krankheiten vorzubeugen (präventive Impfstoffe) oder um etablierte Krankheiten zu behandeln (immuntherapeutische Impfstoffe). Präventive Impfprogramme haben in den letzten ca. 100 Jahren wesentlich zur Reduktion von Infektionskrankheiten beigetragen. Immuntherapeutische Impfstoffe werden erst seit etwa 20 Jahren entwickelt und eingesetzt, etwa gegen persistente Infektionen mit Viren, Bakterien oder Parasiten oder gegen kanzerogene Erkrankungen. Ziel der Impfung ist die Induktion einer zellulären (d.h. im Wesentlichen T- und NK-zellvermittelten) und/oder humoralen (d.h. im Wesentlichen B-zell/antikörper-vermittelten) Immunantwort sowie eines immunologischen Gedächtnisses ("*memory*") gegen antigene Komponenten von Pathogenen oder malignen (tumorgenen) Zellen.

Klassische Vakzine enthalten den gesamten Krankheitserreger in attenuierter (inaktivierter) oder abgetöteter Form, einschließlich dessen genetischen Materials, Nukleinsäuren in Form von DNA oder RNA. Diese klassischen Impfstoffe benötigen für die Herstellung meist besondere Sicherheitsvorkehrungen und/oder die Verwendung von Versuchstieren und/oder die Verwendung von Zellkulturen; zudem müssen sie oft aufwendig und unter der Verwendung von Kühlketten gelagert und transportiert werden. Außerdem bergen sie die Gefahr, dass Stoffe aus der Herstellung (z.B. aus dem Versuchstier oder aus der Zellkultur) im geimpften Individuum Nebenwirkungen erzeugen oder dass es zu unerwünschten Reaktivierungen des Erregers kommt. Probleme existieren auch bei der Diagnostik: So können beispielsweise im Fall der Impfung von Nutztieren geimpfte Tiere nicht von natürlich infizierten Tieren unterschieden werden, so dass das Frühwarnsystem, was auf dem Nachweis von Neuinfektionen basiert, versagen kann. Daher wurden sog. "*subunit*" Vakzine entwickelt, die nur Teile des Erregers enthalten. Voraussetzung dafür ist, dass "Hauptantigene" des jeweiligen Krankheitserregers, bekannt sind. Hauptantigene sind meist Oberflächenbestandteile des Erregers, die vom Immunsystem erkannt werden können, z.B. Proteine einer Virushülle oder des Virus-Kapsids. Diese können auch in Abwesenheit eines kompletten Viruspartikels eine humorale und/oder zelluläre Immunantwort und ein immunologisches Gedächtnis im Wirt gegen das Virus induzieren. Da bei "*subunit* Vakzinierung" also typische Bestandteile des Erregers fehlen, können durch eine Differenzialdiagnose vakzinierte- von natürlich infizierten Individuen unterschieden werden; es wird daher auch von einem "*subunit* Markervakzin" gesprochen. Nachteile vieler *subunit* Vakzine sind eine oft aufwändige Herstellung und eine oft unzureichende Immunogenität: Während die Krankheitserreger selbst effizient (mit den oben ausgeführten Einschränkungen) gezüchtet werden können, müssen deren Hauptantigene durch kostenintensive und meist ineffiziente Verfahren gentechnisch hergestellt und aufwändig gereinigt werden. So gewonnene *subunit* Vakzine sind entsprechend empfindlich, müssen ebenfalls oft gekühlt gelagert und transportiert werden und haben eine geringe Haltbarkeit. Aus diesen Gründen beruht ein Großteil der Massenimpfstoffe immer noch auf dem klassischen Prinzip mit kompletten Krankheitserregern. Beispielsweise basieren die meisten Impfstoffe gegen die weit verbreitete Geflügelkrankheit Infektiöse Bursitis (IBD) derzeit in der Mehrzahl auf attenuierten (abgeschwächten) oder inaktivierten Viren des IBD auslösenden Virus der Infektiösen Bursitis (IBDV).

Das Problem der schwächeren Immunogenität bei *subunit* Vakzinen versucht man durch die zusätzliche Verwendung von Adjuvanzien zu kompensieren. Adjuvanzien sind Stoffe, die sich empirisch als immunstimulierend erwiesen haben. Sie verstärken unspezifisch und oft in wenig verstandener Weise die Immunreaktion. Bislang sind nur wenige Adjuvanzien für den menschlichen Gebrauch zugelassen. Die einzigen Hilfsmittel, die beispielsweise in den USA für den Einsatz am Menschen zugelassen sind, sind Aluminiumsalze, Aluminiumhydroxid und Aluminiumphosphat. Aluminiumsalz-Formulierungen verursachen allerdings zusätzliche Schwierigkeiten bei der Lagerung des betreffenden Vakzins. Außerdem entfalten diese Adjuvanzien nicht mit allen Antigenen eine ausreichend Wirksamkeit.

Die gentechnische Herstellung von Fremdproteinen, zu denen die meisten *subunit* Vakzine zählen, kann in verschiedenen Wirtszellen erfolgen. Neben dem Darmbakterium *Escherichia coli* sind Säugerzellen, die in Zellkulturen vermehrt werden können, Pflanzenzellen und verschiedene Pilze als Wirtssysteme etabliert. Mikrobielle Systeme wie Bakterien und Pilze lassen sich besonders kostengünstig im großen Maßstab züchten. Hefezellen der Hefegattungen *Saccharomyces, Pichia* und *Kluyveromyces* werden bereits seit Dekaden routinemäßig zur Expression von Fremdproteinen eingesetzt. Hefezellen haben gegenüber Bakterien den Vorteil, dass es Eukaryoten sind, d.h. sie gleichen in vielen Aspekten den tierischen Zellen, und eukaryotische Proteine, d.h. Proteine, die in tierischen Zellen gebildet werden und/oder funktionsfähig sein müssen, können in Hefe in nativer oder nahezu nativer Form kostengünstig hergestellt werden (Bathurst, 1994; Gellissen & Hollenberg, 1997). Hefen wurden zunächst nur dafür verwendet die Fremdproteine zu produzieren, und die Proteine wurden aus den Hefezellen gereinigt und als *subunit* Vakzine eingesetzt. Erst seit kurzer Zeit wird versucht, Hefen selbst oder Zellfraktionen der Hefen als Vakzine zu verabreichen.

Seit etwa 5 Jahren wird versucht *Saccharomyces cerevisiae* ("Bäckerhefe", S. *cervisiae*) selbst zur Vakzinierung einzusetzen: So konnte gezeigt werden, dass durch subkutan applizierte, Antigen-exprimierende Zellen von *S*. *cerevisiae* dendritische Zellen aktiviert und Antigen-spezifische T-Zell Immunantworten, speziell zytotoxische T-Zell Antworten gegen bestimmte Antigene erzeugt werden können. Diese zelluläre Immunantwort erwies sich als protektiv gegenüber der Gabe bestimmter Tumorzellen, d.h. in vakzinierten Tieren entstanden im Anschluss an die Vakzinierung weniger Tumoren als in Kontrolltieren. Dieses Verfahren wird derzeit auch in immuntherapeutischen Anwendungen bei Tumorerkrankungen getestet (Stubbs et al., 2001; Lu et al., 2004).

Dem Fachmann sind folgende Quellen aus dem Stand der Technik bekannt, in denen eine Hefe-basierte Vakzinierung beschrieben wird:
Eine Reihe von US Patenten, so z.B. 20090304741, 5830463 und 10738646 und 20070166323 beschreiben die Verwendung von S. *cerevisiae* die mindestens ein rekombinantes Antigen enthalten, in der Immuntherapie. Es wurde gezeigt, dass diese Hefen wirksam sind, um eine Immunreaktion, insbesondere eine Zellvermittelte Immunreaktion, zu stimulieren.

WO 90/15140 offenbart die Immunisierung von Hühnern mit rekombinanten IBDV Antigen. Dieses wurde aus *Kluyveromyces lactis* gewonnen, in der das Antigen exprimiert wurde. WO 90/15140 offenbart jedoch nur *K*. *lactis* Stämme, in denen das IBDV Antigen mittels Plasmidvektoren exprimiert wird, nicht *K*. *lactis* Stämme, in denen das Antigen-exprimierende Gen stabil im Genom integriert ist und induzierbar exprimiert werden kann (Thema der Anmeldung).

WO/2006/044923 offenbart Hefe (*S. cerevisiae)* die verschiedene Proteine des Hepatitis C Virus (HCV) rekombinant exprimiert, und die eine Immunreaktion, vor allem eine T-Zell Antwort, gegen diese HCV Proteine auslösen kann und als Vakzin gegen chronische Hepatitis C eingesetzt werden soll.

WO/2007/092792 beschreibt den möglichen Einsatz rekombinanter *S*. *cerevisiae* gegen Influenzavirus-Infektionen, wobei eine Kombinationen verschiedener Hefestämme benutzt wird, deren Applikation zu einer T-Zell Induktion, also zu einer zellulären Immunantwort führt.

WO/2011/032119 betrifft ein Verfahren zur Verbesserung der Wirksamkeit einer Hefe-basierten Immuntherapie in Patienten. Das Verfahren umfasst ein Hefebasiertes Mittel, dass die Produktion oder das Überleben von CD4+ TH17 Zellen moduliert.

In keinem der zugänglichen Patente wird Hefe nachweislich zur Induktion einer protektiven humoralen Immunantwort gegen Infektionskrankeiten oder Tumoren eingesetzt (Thema dieser Anmeldung).

Wie *S*. *cerevisiae,* so besitzt auch die "Milchhefe" *Kluyveromyces lactis* (*K. lactis)* den GRAS Status (GRAS: *generally regarded as safe*), d.h. sie ist für die Anwendung in Tier oder Mensch geeignet (van Ooyen et al. 2006). Obwohl morphologisch sehr ähnlich der Bäckerhefe *S*. *cerevisiae* haben sich die Entwicklungslinien der beiden Gattungen vor über 100 Millionen Jahren von einem gemeinsamen Vorläufer in unterschiedliche Richtungen entwickelt. *K. lactis* unterscheidet sich daher in vielen Eigenschaften grundlegend von *S. cerevisiae.* Einige dieser Unterschiede sind von großer Bedeutung für die Verwendbarkeit in biotechnologischen Anwendungen. Die Evolution von *S*. *cerevisiae* brachte die Spezialisierung des Stoffwechsels auf alkoholische Gärung mit sich und damit den Verlust vieler Gene der Vorläufer. Die alkoholische Gärung ist für die meisten Hefen allerdings untypisch. Sie erfolgt in *S*. *cerevisiae* bei hohen Glucose-Konzentrationen auch dann, wenn Sauerstoff vorhanden ist und die mitochondriale Atmung eigentlich eine sehr viel effizientere Energieausbeute aus der Zuckerumsetzung zuließe: Die Funktion der Mitochondrien, die "Kraftwerke" der Zelle, wird weitgehend durch "Glucose-Repression" unterdrückt. *K. lactis* unterscheidet sich in der Regulation der Funktion der Mitochondrien erheblich von *S*. *cerevisiae* (Chen and Clark-Walker, 1995, Clark-Walker, 2007). *K*. *lactis* gehört im Gegensatz zu *S*. *cerevisiae* zu den sogenannten "Crabtree negativen" Hefen. Solche Hefen bilden unter strikt aeroben Bedingungen in der Regel kein Ethanol sondern bauen die Glucose über die mitochondriale Aktivität unter Bildung von ATP vollständig zu CO₂ ab. Diese physiologische Eigenschaft ist von fundamentaler Wichtigkeit, da sie zu einer deutlichen Erhöhung der Biomasseausbeute bei Fermentationen im Großmaßstab führt, was eine deutliche Kostensenkung bei der Nutzung dieser Hefen als Produzenten rekombinanter Proteine zur Folge hat. Außerdem haben Studien in *K. lactis* gezeigt, dass Mutationen im Hexokinase-vermittelten Glukose-Signalweg die Expression heterologer Gene verbessern können (Donnini et al., 2004). Eine reduzierte Glukoserepression, insbesondere von respiratorischen Genen, ist ein Merkmal der "Crabtree negativen" Hefen und könnte mit der empirisch beobachteten besseren Fremdgenexpression in solchen Hefen in Zusammenhang stehen.

*K. lactis* und *S*. *cerevisiae* weisen zudem erhebliche Differenzen in der Komposition der Zellwand-Glukane auf (Backhaus et al., 2011); diese Unterschiede beruhen vermutlich auf unterschiedlichen Glycosyltransferasen im Golgi-Apparat, die an der Maturierung von Glykoproteinen beteiligt sind: So enthalten Glykoproteine in *S*. *cerevisiae* vielfach Mannosephosphate, die Glykoproteine in *K. lactis* vor allem terminale N-Acetylglucosamine (Raschke and Ballou, 1972). Es ist anzunehmen, dass diese Unterschiede zwischen *S*. *cerevisiae* und *K. lactis* bei der Glykosylierung und Sekretion von Proteinen sowie in der Zellwandbiosynthese einen erheblichen Einfluss auf die intrazelluläre Lokalisation, die Faltung sowie Stabilität und somit auch auf die Immunogenität von heterolog exprimierten Fremdproteinen haben (Uccelletti et al., 2004).

WO/2010/054649 beschreibt die Herstellung eines rekombinanten Systems von *K*. *lactis.* In den dort angegebenen Anwendungsbeispielen wurden rekombinante Stämme, die vom Stamm *VAK367-D4* abgeleitet waren, zur mukosalen oder oralen Vakzinierung gegen verschiedene Antigene eingesetzt. Nachteil der oralen/muksoalen Vakzinierung ist allerdings, dass die Vakzine in großen Mengen eingesetzt werden müssen, um eine protektive Immunisierung zu erreichen.

### Beschreibung der Abbildungen

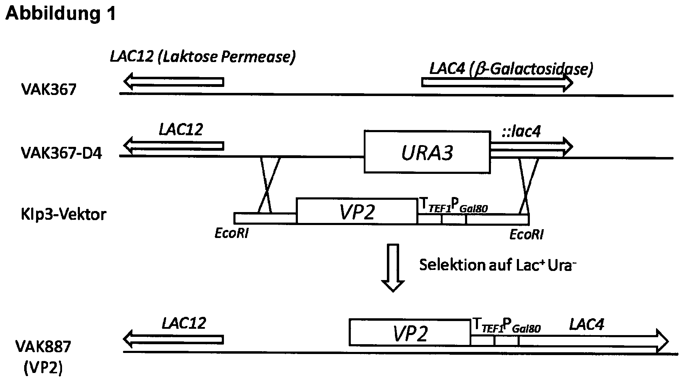

**Abb. 1** zeigt schematisch die Herstellung des Impfstammes VAK887, der das IBDV VP2 Fremdgen trägt, über homologe Rekombination in den VAK367-D4 Ausgangsstamm. Über Transformation des Plasmids Klp3-MCS (SEQ ID Nr.: 10), das das VP2 Gen des IBDV Stammes D78 enthielt, wurde das VP2 Fremdgen über homologe Rekombination. in den chromosomalen LAC4 Genort eingesetzt, der durch Insertion des *URA3* Gens zerstört war. Bei der Rekombination in das Wirtsgenom wurde das *URA3* Gen durch das VP2 Gen ersetzt und das *LAC4* Gen wieder hergestellt; rekombinante Hefestämme konnten durch Selektion auf Lactose-Medium ohne Uracil erhalten werden. Nachfolgend wird die Expression von *LAC4* (β-Galaktosidase) über den *KIGAL80* Promoter, die Expression des VP2 Gens über den LAC4 Promoter gesteuert.

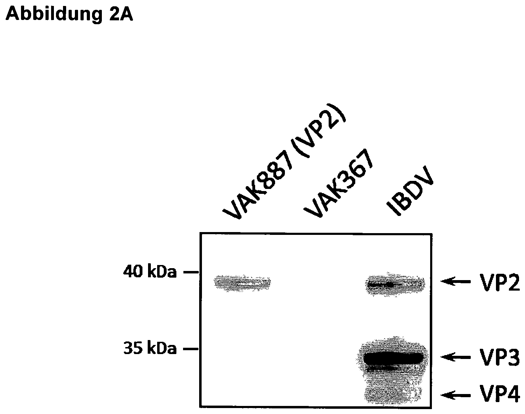

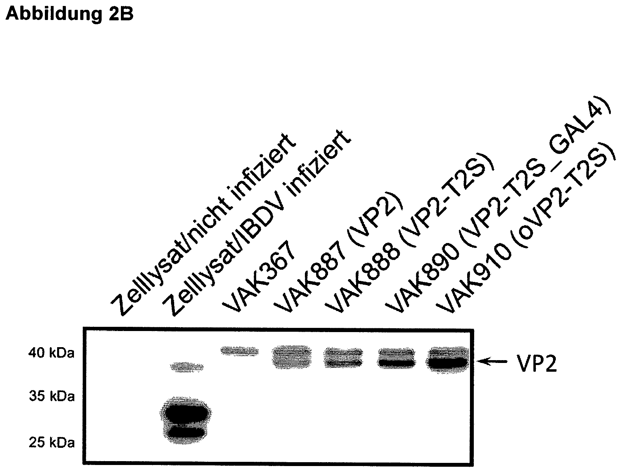

**Abb. 2A** zeigt die Expression von IBDV VP2 durch den Stamm VAK887 im Vergleich zum Ursprungsstamm (VAK367) und im Vergleich zu IBDV infizierten Hühnerzellen mittels Western Analyse mit einem VP2-spezifischen Antikörper. **Abb. 2B** zeigt die Expressionsanalyse von rekombinantem IBDV VP2 bzw. mutiertem IBDV VP2-T2S in verschiedenen VP2 exprimierenden *K. lactis* Varianten. Die ursprüngliche *K. lactis* Variante VP2 (VAK887) exprimierte nur moderate Mengen an viralem Protein. Die VP2 Proteinexpression konnte im Stamm *K. lactis* VP2-T2S (VAK888) gesteigert werden, indem Threonin an Aminosäureposition 2 des VP2 Proteins gegen Serin ausgetauscht wurde. Eine weitere Steigerung konnte erzielt werden durch Erhöhung der *KIGAL4* Gendosis (VP2-T2S_GAL4 = VAK890) bzw. durch Verwendung eines Hefe-Codon optimierten synthetischen VP2 Gens (oVP2-T2S = VAK910).

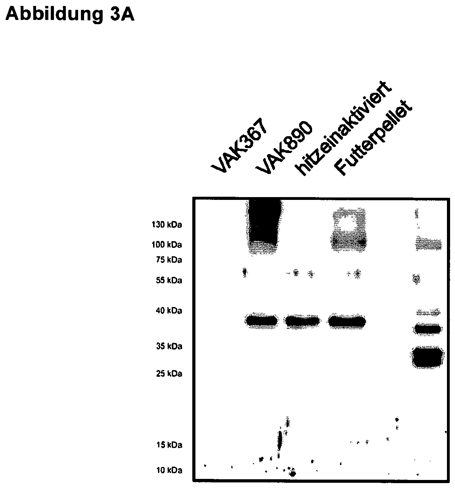

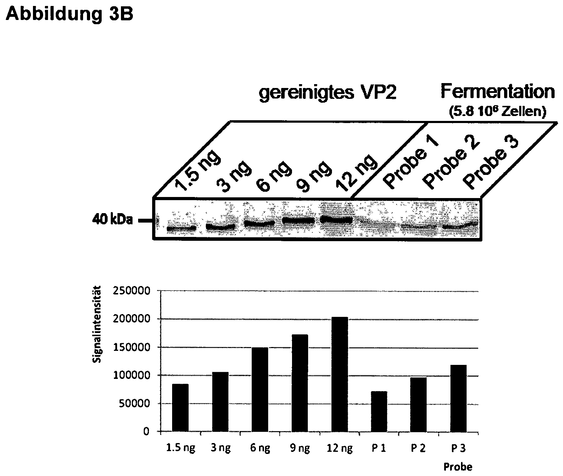

**Abb. 3** zeigt, dass die Hitzeinaktivierung der erfindungsgemäßen Hefen bei 90° C für 2 Stunden nicht zu einem Verlust des rekombinanten VP2-T2S Proteins führt (Abb. 3A). Es wurden jeweils gleiche Proteinmengen aus nicht inaktivierter Hefe, inaktivierter Hefe und Hefe aus einem Futterpellet auf einem SDS PAGE aufgetrennt und im Vergleich zu Zelllysaten aus Geflügelzellen, die mit IBDV infiziert oder nicht infiziert waren, in einem Western-Blot mit einem anti-VP2 Antikörper getestet. Abb3 zeigt weiterhin, dass die Menge an VP2-T2S in der Variante VAK890 ca. 0,7 fg heterologes Protein pro Hefezelle beträgt (Abb. 3B). Hier wurden definierte Mengen von gereinigtem VP2-T2S im Vergleich zu VP2 aus einer definierten Zellzahl im Fermenter gezogener *K*. *lactis* (Stamm VAK890) im Western Blot angefärbt und das Ergebnis densitometrisch ausgewertet.

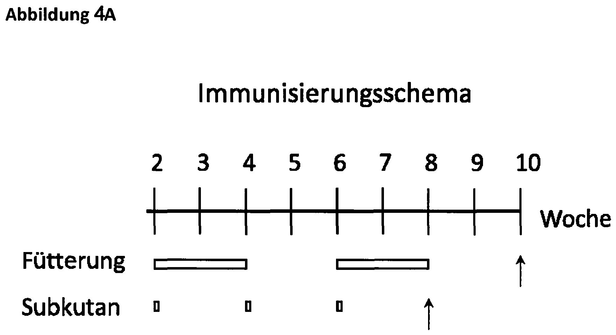

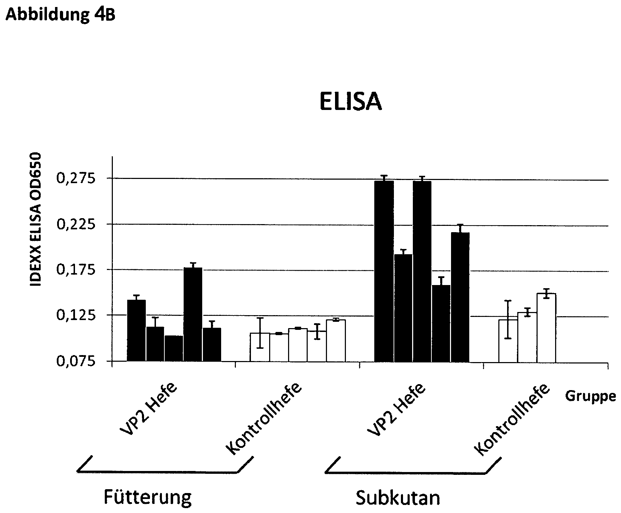

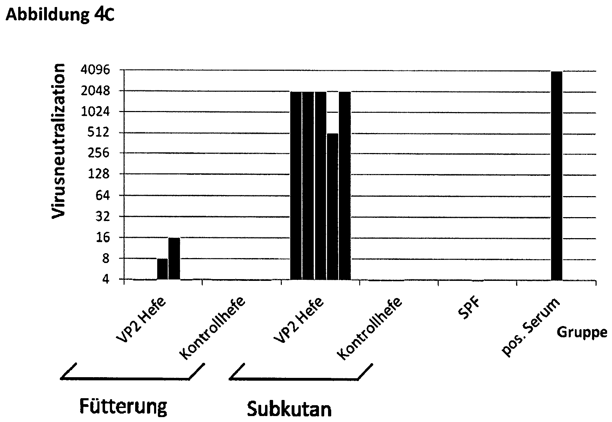

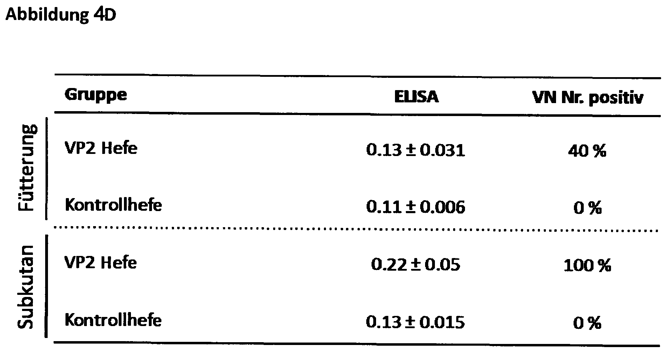

**Abb. 4** erläutert die Vakzinierung in Mäusen mit subkutan applizierten, hitzeinaktivierten, kompletten Hefezellen der *K*. *lactis* Variante VAK890 im Vergleich zur oralen Vakzinierung mit kompletten Hefezellen der *K*. *lactis* Variante VAK890. Abb. 4A zeigt das Immunisierungsschema: Es wurde dreimal subkutan immunisiert, mit je zwei Wochen Pause; im Vergleich wurde zweimal zwei Wochen lang gefüttert. Zwei Wochen (Pfeil) nach der letzten Hefeapplikation wurden Serumproben aus den behandelten Mäusen in einem IBDV-spezifischen ELISA (Abb. 4B) und in einem IBDV-Neutralisationsassay auf Anwesenheit von Anti-VP2 Antikörper geprüft (Abb. 4C). Abb. 4D fasst zusammen, dass Mäuse, die mit VP2-exprimierender *K*. *lactis* (Stamm Kl VP2-T2S_GAL4 (VAK890)) behandelt wurden, deutlich höhere Titer an Antikörpern/neutralisierenden Antikörpern aufweisen als Mäuse, die mit *K*. *lactis* Wildtyp (Stamm VAK367) behandelt wurden. Zudem wurde gezeigt, dass subkutan applizierte *K. lactis* (Stamm VAK890) deutlich höhere Titer an Antikörpern/ neutralisierenden Antikörpern aufweisen, als Mäuse, die mit *K. lactis* (Stamm VAK890) gefüttert wurden. Mäuse, die oral mit *K*. *lactis* (Stamm 890) immunisiert wurden, zeigten allerdings auch einen erhöhten Titer an Antikörpern/neutralisierenden Antikörpern im Vergleich zu Mäusen, die mit *K*. *lactis* Wildtyp (Stamm VAK367) behandelt wurden.

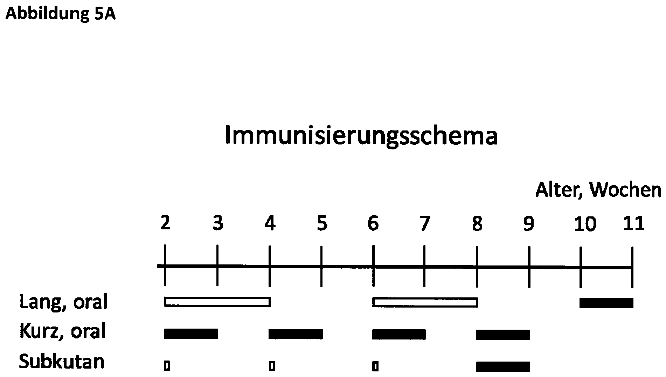

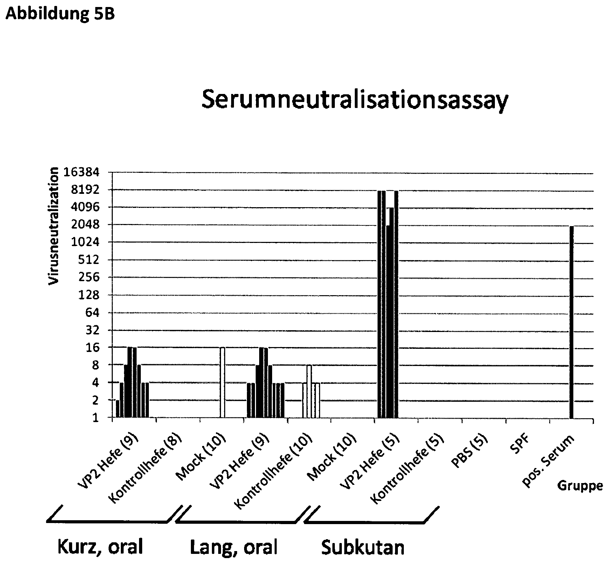

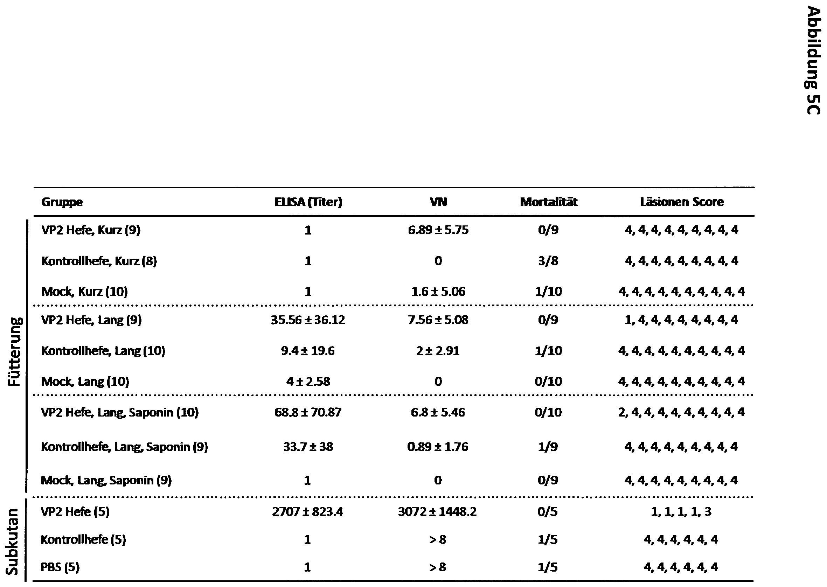

**Abb. 5** zeigt die orale und subkutane Vakzinierung in Hühnern mit hitzeinaktivierten, kompletten Hefezellen der *K*. *lactis* Variante VP2-T2S_GAL4 (VAK890). Für die orale Vakzinierung wurde entweder ein kurzes 1/1/1/1/1 Schema (1 Woche füttern, 1 Woche Pause, 1 Woche füttern, usw.) oder ein längeres 2/2/2 Schema angewandt (Abb. 5A). Nach 1 Woche bzw. 2 Wochen Pause nach der Vakzinierung (Abb. 7A), wurden alle behandelten Tiere mit IBDV (Stamm Edgar) in einer Konzentration von 100 EID50 pro Tier infiziert (Virus *challenge;* schwarze Balken). Nach oraler Vakzinierung, insbesondere nach Anwendung des verlängerten Behandlungsschemas, konnten in mehreren Tieren erhöhte Titer an Virus-neutralisierenden Antikörpern nachgewiesen werden. Die subkutane Vakzinierung mit rekombinantem *K. lactis* hingegen erzeugte hohe Titer an Virus-neutralisierenden Antikörpern in allen behandelten Tieren (Abb. 5B, C; IBDV-spezifischer ELISA, IBDV-Neutralisationsassay). Keines der mit rekombinanter *K. lactis-Hefe* behandelten Tiere starb nach Infektion mit IBDV, unabhängig davon, welches Behandlungsschema angewendet wurde. Im Gegensatz dazu betrug die Sterblichkeitsrate in der Kontrollgruppe 10-35% (Abb. 5C). Die Analyse der Läsionen in den Bursae der behandelten Tiere zeigte, dass etwa 10% der oral behandelten Tiere nach Anwendung des verlängerten Behandlungsschemas keine Anzeichen einer Virusinfektion nach Inokulation mit IBDV aufwiesen: Dabei wurde ein sog. "Läsionen score" verwendet - score 1, 2 indizieren nicht oder kaum beschädigte Bursae; score 3, 4 indizieren beschädigte und stark beschädigte Bursae. Im Gegensatz dazu zeigten alle Tiere, in denen die rekombinante *K. lactis* Stamm VAK890 subkutan appliziert wurde, einen vollständigen Impfschutz gegen IBDV (Abb. 5C).

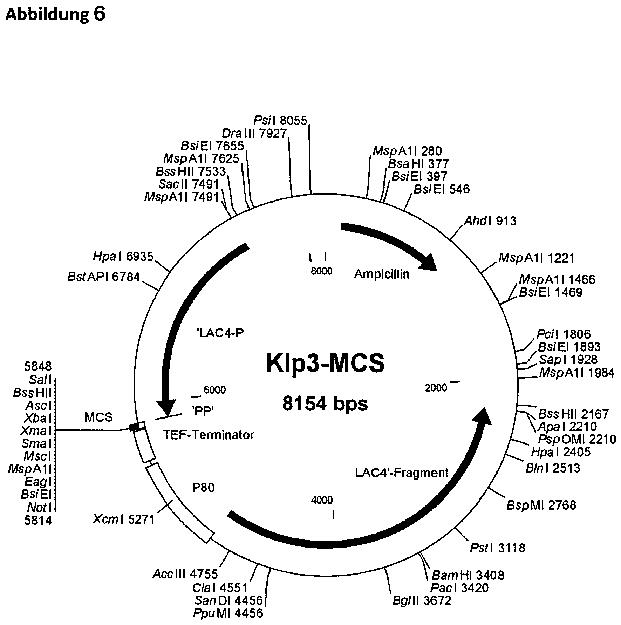

**Abb. 6** zeigt schematisch den Aufbau des Vektors Klp3-MCS **(SEQ ID Nr.:10).**

### Beschreibung der Erfindung:

Die Möglichkeit der Anwendung rekombinanter Hefen zur subkutanen Vakzinierung ist dem Fachmann aus dem Stand der Technik bekannt: Stubbs et al., (2001) Nat. Med. 7: 625-629; Stubbs and Wilson (2002) Curr. Opin. Mol. Ther. 4: 35-40; Wansley et al., (2008) Clin. Cancer Res. 14: 4316-4325; US 5,830,463, WO/2006/044923; WO/2007/092792 und WO/2011/032119. In den Ausführungsbeispielen dieser Publikationen wurde allerdings ausschließlich mit der Hefe *Saccharomyces cerevisiae* gearbeitet. "Hefe" ist ein Sammelbegriff für einzellig wachsende eukaryotische Mikroorganismen mit z.T. sehr unterschiedlichen Eigenschaften aufgrund divergenter Evolution über hunderte Millionen von Jahren (ca. 100 Mio Jahre für *S*. *cerevisiae* und *K. lactis*). Beim Einsatz von *S*. *cerevisiae* und *K*. *lactis* zum Zwecke der Vakzinierung in höheren Eukaryoten wie Tier oder Mensch ist daher davon auszugehen, dass sich eine von *S*. *cerevisiae* ausgelöste Immunantwort stark von einer von *K. lactis* ausgelösten Immunantwort unterscheidet. Dies gilt sowohl für die Immunantwort gegen in der Hefe exprimierten Fremdantigene als auch für die Immunantwort gegen hefeeigene Antigene. Bei subkutanen Immunisierungen mit kompletten *S*. *cerevisiae* Zellen wurde eine T-Zell Induktion, also eine *zelluläre* Immunantwort erzeugt. Eine protektive, *humorale* Immunantwort gegen ein Antigen mit rekombinanter *S*. *cerevisiae* auf einfachem Wege (d.h. durch direkte Applikation eines einzelnen Antigen-exprimierenden Stammes) wurde bisher im Stand der Technik nicht belegt.

Ausgehend von dem oben dargestellten Hintergrund stellte sich die Aufgabe, ein Verfahren bereitzustellen, mit dem eine protektive, humorale Immunantwort gegen das VP2-Antigen des Virus der Infektiösen Bursitis (IBDV)erzeugt werden kann. Eine weitere Aufgabe bestand darin, ein *subunit* Markervakzin herzustellen, mit dem es möglich ist, vakzinierte gegenüber natürlich infizierten Individuen zu unterscheiden. Eine weitere Aufgabe bestand darin, ein *subunit* Markervakzin herzustellen, das zugleich stark adjuvierende Eigenschaften aufweist und damit stark immunogen ist. Diese Aufgaben wurden gelöst durch Bereitstellung einer rekombinanten Hefe der Spezies *Kluyveromyces lactis,* die ein Gen, das für ein VP2-Antigen des Virus der Infektiösen Bursitis (IBDV) codiert, als Fremdgen trägt, das in das Hefegenom integriert ist, und die die Expression des VP2-Antigens des Virus der Infektiösen Bursitis (IBDV) als Fremdprotein ermöglicht, dadurch gekennzeichnet, dass dieser *Kluyveromyces lactis* Stamm ausgewählt ist aus:
*Kluyveromyces lactis* DSM 25405,
*Kluyveromyces lactis* DSM 25406, und
*Kluyveromyces lactis* DSM 25407.

Der zur Herstellung dieser Stämme verwendete Ausgangsstamm VAK 367-D4 (DSM 23097) erlaubt die gezielte Integration des Gens für die Expression des VP2-Antigens des Virus der Infektiösen Bursitis (IBDV) als Fremdgen in das Hefegenom. Rekombinante Hefen, die dieses Fremdgen exprimieren, können mit diesem System schnell (d.h. innerhalb weniger Wochen) hergestellt werden. Die Hefen können im Fermenter in großen Quantitäten (z.B. Kilogramm (kg) Bereich) kostengünstig vermehrt werden. Durch regulierte Expression und Fermentation im Fed-Batch Verfahren können auch zytotoxische Antigene in diesem Hefesystem exprimiert werden. Nach Expression des Fremdgens wird die Hefe hitzeinaktiviert und kann dann als Pulver ungekühlt gelagert und transportiert werden. Das Hefepulver kann direkt, d.h. ohne weitere Fraktionierung, entweder als Emulsion oder als Pellet (siehe Ausführungsbeispiele) als *subunit* Markervakzin eingesetzt werden. Die Antigen-Formulierung und der für die effektive, d.h. protektive Immunisierung notwendige Adjuvanzeffekt werden durch zwei Faktoren gewährleistet: (i) durch die Möglichkeit der gezielten gentechnischen Modifikation des exprimierten Fremdproteins, (ii) durch die Expression des Fremdproteins in der Hefe und die direkte Anwendung der Hefe in oraler oder subkutaner Form; die Hefe selbst hat einen starken Adjuvanzeffekt. Bevorzugt ist die subkutane Applikation. Es wurde ein rekombinanter Hefestamm hergestellt; dieser exprimiert ein spezifisches Virusantigen und kann in dem erfindungsgemäßen Verfahren zur subkutanten Vakzinierung verwendet werden. Es wurde eine vollständige präventive Protektion gegen eine Infektion durch das betreffende Virus erreicht. Dabei wurden nur sehr geringe Mengen an Hefe (z.B. im Milligramm (mg) Bereich bei subkutaner Anwendung in Geflügel) eingesetzt werden. Es war nur eine 2-3 malige Anwendung notwendig, um diese Protektion zu erreichen. Das erfindungsgemäße Verfahren ist sowohl für die Anwendung im humanmedizinischen als auch veterinärmedizinischen Bereich geeignet. Bevorzugt ist die Anwendung des erfindungsgemäßen Verfahrens im veterinärmedizinischen Bereich.

Das erfindungsgemäße Verfahren wird mit der Hefe *Kluyveromyces lactis* durchgeführt.

Die Hefe *K. lactis* gehört zu den sogenannten "food grade" Hefen, die den GRAS Status (GRAS: generally regarded as safe) haben. Wie die Bäckerhefe, die als Nahrungsmittelzusatz über Jahrtausende erprobt und bewährt ist, gilt auch die in Molkereiprodukten häufig vertretene Hefe *K*. *lactis* für die Lebensmittelindustrie als unbedenklich.

Neben der unter "Stand der Technik" erläuterten Möglichkeit zur Fermentation weist die Hefe *K. lactis* gegenüber *S*. *cerevisae* zahlreiche Vorteile im Hinblick auf die Expression heterologer Gene auf. *K. lactis* gehört zu den sogenannten "petite negative" Hefen, das heißt, der Verlust der mitochondrialen DNA ist letal (aufgrund des Zusammenbruchs des mitochondrialen Membranpotentials (Chen et al., 1995; Clark-Walker, 2007)). Die Mitochondrienfunktion ist eng gekoppelt an Ca²⁺- abhängige Signalvermittlung, die Produktion von reaktiven Sauerstoffverbindungen, die Stressantwort der Zelle, die Proteinglykosylierung und die Zellwandintegrität. Dadurch beeinflusst die Mitochondrienfunktion entscheidend die Produktion von rekombinanten Glycoproteinen und die Zusammensetzung der Zellwand.

Bei Hefen und Säugern sind die ersten Schritte der N-Glycosylierung von Proteinen, die im Endoplasmatischen Retikulum stattfindet, gleich. Allerdings weichen die im Golgi Apparat stattfindenden Schritte voneinander ab. Die im Golgi Apparat vorhandenen Glycoslytransferasen sind in den unterschiedlichen Hefespezies verschieden. Dadurch kommt es zu Unterschieden in der Zusammensetzung der Glycoproteine in der Zellwand. In *K. lactis* weisen die Glycoproteine terminale N-Acetylglucosamine auf, im Gegensatz zu Mannosephosphat bei *S*. *cerevisiae.* (Raschke und Ballou, 1972). Dies könnte bei Vakzinierungen erhebliche Auswirkungen auf die Stimulierung des Immunsystems durch die jeweilige Hefespezies haben.

Die verbesserte Sekretion rekombinanter Proteine in *K. lactis* Mutanten mit veränderter α1,6-Mannosyltransferase (*KlOCH1*) verdeutlicht die Verbindung zwischen Proteinglycosylierung/ Sekretion und Zellwandbiosynthese (Uccelletti et al., 2004). Veränderungen in der Proteinglycosylierung beeinflussen außerdem die intrazelluläre Lokalisation rekombinanter Proteine, die auf dem Weg zur Sekretion aufgrund fehlerhafter Faltung zurückgehalten werden.

*K. lactis* ist eine der wenigen Hefearten, die Laktose als Kohlenstoff- und Energiequelle verwerten können. Laktose ist ein billiger Zucker, der als Bestandteil der Molke in hohen Mengen (z.B. als Beiprodukt im der Molkereiwirtschaft) anfällt. *K*. *lactis* kann mit Laktose ähnliche Wachstumsraten wie mit Glukose erreichen. Die Regulation der am Laktose-Stoffwechsel beteiligten Gene wurde intensiv untersucht. Der starke β-Galaktosidasepromoter (*LAC4*) kann zur Regulierung der Expression heterologer Gene und Produktion rekombinanter Proteine benutzt werden. (van Ooyen et al., 2006, Breunig et al., 2000). Aufgrund der verminderten Glukoserepression kann die heterologe Expression von Genen in *K. lactis* Kulturen, die in Glukose-haltigem Medium kultiviert wurden, schnell und effizient durch die Zugabe von Laktose induziert werden.

Erfindungsgemäß wurden über gentechnische Verfahren die in Anspruch 1 genannten *K. lactis* Stämme, die Varianten des Stammes *VAK367-D4* darstellen" generiert, die die gezielte Integration von Fremdgenen am *LAC4* Locus des Hefegenoms erlauben **(****Abb. 1****).** Diese Integration erfordert nur einen Schritt über ein entsprechend konstruiertes Plasmid; eine Selektion von rekombinanten Stämmen ist ohne die Verwendung von Antibiotika-Resistenzgenen möglich, und die Fremdgenexpression in den rekombinanten Stämmen kann über den *LAC4* Promoter durch die Zugabe von Laktose in das Medium induziert werden. *K. lactis* Zellen mit integrierten Fremdgenen können über diese Methode in wenigen Wochen generiert und charakterisiert werden. Beide Aspekte dieses Systems sind von großer Wichtigkeit: Zum einen ist so die reproduzierbare Anzucht von Hefezellen möglich, die jeweils definierte Mengen eines Fremdproteins enthalten **(****Abb. 2****,** **3****).** Durch die zusätzliche Integration von Genen des *KlGal4* Transaktivators in das Hefegenom kann zudem die Expressionsrate des Fremdgens signifikant gesteigert werden (Kuger et al., 1990).

In einer weiteren Ausführungsform betrifft die Erfindung die in Anspruch 1 genannten Abkömmlinge des *K*. *lactis* Stamms *VAK367-D4* zur Verwendung in einem Verfahren zur subkutanen Vakzinierung. Es wurde eine Serie (VAK) auf dem *K. lactis* Stamm *VAK367-D4* aufbauender rekombinanter Varianten generiert. Generell exprimieren diese Varianten induzierbar signifikante Mengen eines Fremdproteins, oder Domänen dieses Fremdproteins, oder Domänen dieses Fremdproteins fusioniert mit artfremden Proteindomänen. Die dabei verwandten artfremden Proteindomänen dienen der gezielten Stimulation der Immunantwort (Adjuvanz) bzw. der gezielten Kompartimentierung des exprimierten Fremdproteins in der Hefezelle. Neben Adjuvanzeffekten ist die Kompartimentierung des exprimierten Fremdproteins wichtig für die Optimierung der Expression bzw. die Formulierung des Expressionsproduktes.

In einer weiteren Ausführungsform wird das erfindungsgemäße Verfahren durchgeführt mit Abkömmlingen von *VAK367-D4* zur Verwendung als *subunit* Markervakzin. Die Verwendung rekombinanter *K*. *lactis,* die nur definierte Proteinantigene (Fremdproteine) exprimieren, als Vakzin erlaubt in einer Differenzialdiagnose die Diskriminierung vakzinierter gegenüber natürlich infizierten Individuen. Einer dieser rekombinanten *K. lactis* Stämme (siehe Ausführungsbeispiele) wurde erfolgreich zur oralen und subkutanten Impfung eingesetzt. Bei der subkutanen Anwendung wurde eine vollständige Protektion der Impflinge erhalten.

Als "Fremdprotein" im Sinne dieser Erfindung ist das VP2-Antigen des Virus der Infektiösen Bursitis (IBDV) gemeint, das geeignet ist, eine protektive Immunantwort, bevorzugt eine protektive humorale Immunantwort, im Menschen oder in einem Tier gegen ein Pathogen zu erzeugen. In der am meisten bevorzugten Ausführungsform der Erfindung stammen die Fremdproteine von Vertretern der Familie *Birnaviridae,* wie z. B. dem IBD-Virus, und sind in der Lage, eine protektive Immunantwort, vorzugsweise eine protektive humorale Immunantwort zu induzieren.

In einem Beispiel wurde eine *K. lactis VAK367-D4* Variante VP2 (VAK887) erzeugt, die als Fremdprotein das Capsid-formierende VP2-Antigen des Virus der Infektiösen Bursitis (IBDV Stamm D78) exprimiert **(SEQ ID NR.: 1 und 2).** Besonders bevorzugt ist eine *K. lactis VAK367-D4* Variante VP2-T2S (VAK888), bei der das VP2 Protein an Aminosäureposition 2 mutiert war (Austausch Threonin gegen Serin; Jagadish et al. (1991)) und die die Nukleotid- bzw. Aminsosäursequenz gemäß **SEQ ID NR.: 3 und 4** aufweist.

In einer besonders bevorzugten Ausführungsform der Erfindung wurde eine optimierte *K. lactis VAK367-D4* Variante, VP2T2S_GAL4, erzeugt, bei der das VP2 Protein an Aminosäureposition 2 mutiert war **(SEQ ID NR.: 3 und 4)** und die zusätzlich mindestens zwei *KIGAL4* Gene enthielt (VAK890). Besonders bevorzugt ist eine *K. lactis VAK367-D4* Variante, oVP2-T2S, in der das mutierte VP2-Antigen durch die Hefe Codon-optimierte Nucleotidsäuresequenz mit der **SEQ ID NR.: 5** codiert wird bzw. in der das rekombinant exprimierte mutierte VP2 Antigen die Aminosäursequenz gemäß **SEQ ID NR.: 6** aufweist. Die optimierte *K. lactis* VP2-T2S_ GAL4 Variante (VAK890) weist folgende Vorteile auf:
- Durch die Mutation wurde das Fremdprotein zusätzlich stabilisiert.
- Durch die Überexpression des Trans-Aktivators und/oder durch Codon-Optimierung der Sequenz konnte eine deutliche Steigerung der VP2 Expression erreicht werden **(****Abb. 2****).**
- Die Integration zusätzlicher *KIGAL4* Gene korrelierte auch mit einer höheren Wachstumsrate dieser *K*. *lactis* Variante.
- Diese *K. lactis* Variante weist eine besonders hohe Reproduzierbarkeit in der Hochzelldichtefermentation und der Menge an exprimiertem VP2 Protein auf **(****Abb. 3****).**

Die erfindungsgemäß erzeugte *K. lactis* VP2-T2S_GAL4 Variante, die als Fremdprotein das mutierte VP2-Antigen des IBDV rekombinant exprimiert sowie weitere Kopien des *KIGAL4* Transaktivatorgens enthält (VAK890), wurde am 29. November 2011 bei der Deutsche Sammlung von Mikroorganismen und Zellkulturen GmbH, DSMZ, Inhoffenstrasse 7B, 38124 Braunschweig, Deutschland, entsprechend dem Budapester Vertrag unter der Nummer DSM 25405 hinterlegt.

Die erfindungsgemäß erzeugte *K. lactis* oVP2-T2S Variante, die als Fremdprotein das mutierte und Codon-optimierte VP2-Antigen des IBDV rekombinant exprimiert (VAK910), wurde am 29. November 2011 bei der Deutsche Sammlung von Mikroorganismen und Zellkulturen GmbH, DSMZ, Inhoffenstrasse 7B, 38124 Braunschweig, Deutschland, entsprechend dem Budapester Vertrag unter der Nummer DSM 25406 hinterlegt.
Die erfindungsgemäß erzeugte *K. lactis* oVP2-T2S Variante, die als Fremdprotein das mutierte und Codon-optimierte VP2-Antigen des IBDV rekombinant exprimiert sowie weitere Kopien des *KIGAL4* Transaktivorgens enthält (VAK911), wurde am 29. November 2011 bei der Deutsche Sammlung von Mikroorganismen und Zellkulturen GmbH, DSMZ, Inhoffenstrasse 7B, 38124 Braunschweig, Deutschland, entsprechend dem Budapester Vertrag unter der Nummer DSM 25407 hinterlegt

Eine weitere Ausführungsform betrifft die Verwendung der erfindungsgemäßen rekombinanten Hefen in einem Verfahren zur Erzeugung einer protektiven Immunisierung, insbesondere einer protektiven humoralen Immunisierung.
Ein solches Verfahren umfasst folgende Schritte:
a) Anzucht und Vermehrung der erfindungsgemäßen rekombinanten Hefen,
b) Ernte und Inaktivierung der Hefen,
c) Applikation der rekombinanten Hefen gemäß einem festzulegenden Immunisierungsschema,
d) Titerbestimmung der gebildeten Antikörper und/oder
e) Nachweis der Immunisierung.

Die Anzucht und Vermehrung der erfindungsgemäßen rekombinanten Hefen kann mit jedem konventionell verfügbaren Verfahren erfolgen. Besonders bevorzugt sind dabei Verfahren, die kostengünstig zu hohen Zellausbeuten führen. Dazu gehören Fermentationsverfahren, insbesondere Verfahren der Hochzelldichtefermentation. Als besonders vorteilhaft hat sich die Durchführung der Fermentation unter Anwendung eines *fed-batch* Fermentationsprotokolls erwiesen.

In einer bevorzugten Ausführungsform wird die protektive, humorale Immunisierung dadurch erreicht, dass die rekombinanten Hefen oral/mukosal oder subkutan appliziert werden. In einer besonders bevorzugten Ausführungsform der Erfindung werden die rekombinanten Hefen subkutan appliziert. Insbesondere bevorzugt in dem erfindungsgemäßen Verfahren ist die Verwendung der in Anspruch 1 genannten *K. lactis* Stämme für die subkutane Applikation.

Die rekombinanten Hefezellen sollten in dem erfindungsgemäßen Verfahren inaktiviert/abgetötet eingesetzt werden. Dazu werden die Hefe nach der Anzucht und Expression der Fremdgene getrocknet und anschließend inaktiviert. Die Inaktivierung kann mit jedem konventionell verfügbaren Verfahren durchgeführt werden. Besonders geeignet zur Anwendung in dem erfindungsgemäßen Verfahren ist die Hitzeinaktivierung (z.B. Hitzeinaktivierung für 2 Stunden bei 90°C).

Für die orale/mukosale Vakzinierung kann beispielsweise ein kurzes 1/1/1/1 Immunisierungsschema (1 Woche füttern, 1 Woche Pause, 1 Woche füttern, usw.) oder ein längeres 2/2/2 Schema (2 Wochen füttern, 2 Wochen Pause, 2 Wochen füttern, usw.) angewandt werden. Für die subkutane Vakzinierung kann beispielsweise eine zweifache oder dreifache Anwendung im Abstand von je zwei Wochen verwendet werden **(****Abb. 4** **und** **5****)**

Zum Nachweis der erfolgten Immunisierung stehen alle konventionellen Methoden zur Verfügung. In einer Ausführungsform der Erfindung wird zum Nachweis der Immunisierung der Titer an Virus-neutralisierenden Antikörpern getestet. Dazu können zum Beispiel spezifische ELISA-Tests oder Neutralisationsassays durchgeführt werden. Im Neutralisationsassay wird eine definierte Anzahl an IBD-Viren mit einer definierten Menge Serum eines immunisierten Tieres oder eines Kontrolltieres versetzt. Nachfolgend wird auf eine Inhibition der Infektion (Neutralisation) durch die derart behandelten Viren in Zellkultur getestet. Ob eine Immunisierung erfolgreich war, kann auch in einem "*challenge*" Experiment, z.B. in einem "Viruschallenge" Experiment geprüft werden. Dazu wird den behandelten Tieren eine Dosis eines pathogenen Mikroorganismus oder Virus verabreicht, die normalerweise bei nicht immunisierten Tieren zur Erkrankung führen würde. Erfolgt nach einem solchen *challenge* keine Erkrankung der Tiere, ist der Nachweis für die erfolgreiche Immunisierung erbracht **(****Abb. 5****).** Schließlich kann der Nachweis der Immunisierung auch durch Immunhistochemie erbracht werden. Dabei werden nach dem *challenge* die Zielorgane des Pathogens auf Infektion oder Läsion hin untersucht **(****Abb. 5****).**

Erfindungsgemäß wurde gezeigt, dass rekombinante *K. lactis* Varianten, die jeweils von *VAK367-D4* abgeleitet wurden, erfolgreich zur Vakzinierung durch subkutane Applikation eingesetzt werden konnten. Die in den Ausführungsbeispielen ausgeführte Stamm-Variante VAK890 exprimiert das VP2-Antigen des Virus der Infektiösen Bursitis (IBDV; Stamm D78). Beim VP2 des IBDV handelt es sich um ein virales Capsid-formierendes Protein. Für VP2 ist bekannt, dass das Auslösen einer humoralen Immunantwort gegen dieses Antigen hinreichend ist, um einen infizierten Organismus präventiv vor einer nachfolgenden Infektion durch das betreffende Virus (IBDV) zu schützen. Das Auslösen einer wirksamen humoralen Immunantwort konnte einerseits über die Quantifizierung Virus-neutralisierender Antikörper indiziert werden. Andererseits wurde der Nachweis einer protektiven Immunantwort über ein "Virus-*challenge*-Experiment" und eine Immunhistochemie nach dem Virus-*challenge* geführt. Erfindungsgemäß konnte so rekombinante *K. lactis,* bzw. rekombinante *K*. *lactis,* ausgehend vom Stamm *VAK367-D4,* in subkutanen Anwendungen als effektiv wirksames, d.h. zu 90-100% protektives Vakzin (90-100% entspricht "Gold-Standard" bei der Vakzinierung) etabliert werden **(****Abb. 4** **und** **5****).** Die rekombinante *K. lactis,* bzw. rekombinante *K. lactis,* ausgehend vom Stamm *VAK367-D4,* wurde somit als "*subunit*"-Markervakzin gegen Infektionserreger wie z.B. Viren etabliert. Das heißt, als Antigen wurde eine einzelne, immunogene Proteinuntereinheit eines Virus verwendet. Der Einsatz als "*subunit*" Markervakzin impliziert, dass dessen Verwendung die Unterscheidung vakzinierter von nicht-vakzinierten, infizierten Organismen ermöglicht. Dies ist z.B. möglich über die Verwendung einer differenziellen Diagnosemethode, die sowohl Antikörper gegen das zur Vakzinierung verwendete Antigen, als auch Antikörper gegen ein weiteres Antigen des Infektionserregers nachweist. Durch die Immunisierung mit dem rekombinanten *K*. *lactis* Stamm VAK890, ausgehend vom Stamm *VAK367-D4,* konnten hohe Antikörpertiter gegen das entsprechende Virusantigen erzeugt werden. Für diese Antikörper konnte gezeigt werden, dass sie virusneutraliserend sind. Bereits über diese Eigenschaft und den gemessenen hohen Titer kann empirisch ableitet werden, dass diese humorale Immunantwort ausreicht, um einen Organismus vor einer nachfolgenden Infektion mit dem betreffenden Virus zu schützen. Der finale Beweis konnte für das IBDV erbracht werden. Der hohe Titer erzeugter virusneutralsierender Antikörper korrelierte im Huhnmodell mit einer vollständigen Protektion der vakzinierten Tiere gegen eine nachfolgende Virusinfektion (Abb. 5).

Die Verwendung der in Anspruch 1 beanspruchten *K. lactis* Stämme, die gentechnisch veränderte Varianten des Stammes *VAK367-D4* darstellen, wie zum Beispiel *K. lactis* VP2-T2S_GAL4 (VAK890), hat folgende, wesentliche Vorteile gegenüber herkömmlichen Verfahren:
1. Für die Verwendung zur Fremdgenexpression hat *K. lactis* gegenüber *S*. *cerevisiae* wesentliche, grundsätzliche Vorteile, die in der über Jahrmillionen von *S*. *cerevisae* divergierenden Physiologie von *K. lactis* begründet sind.
2. Die Expression des Fremdgens erfolgt nicht über Plasmidvektoren, wie in WO 90/15140 beschrieben, sondern nach gezielter und stabiler Integration des Fremdgens in einen definierten Genort des *K. lactis* Genoms. Dies erlaubt eine hohe Reproduzierbarkeit der Proteinexpression unter nicht-selektiven Bedingungen. Dieser Aspekt ist wesentlich für die reproduzierbare Erzeugung des Impfstoffs durch Kultivierung des Hefestammes im Fermenter. Das Prinzip des Stammes *VAK367-D4* und Abkömmlingen hiervon ist bereits für eine orale Vakzinierung beschrieben worden (WO 20101054649 A2). In der vorliegenden Erfindung wird nun gezeigt, dass der Stamm *VAK367-D4* und seine Abkömmlinge, insbesondere *K. lactis* VP2-T2S_GAL4, bei subkutaner Vakzinierung unter Einsatz wesentlich geringerer Hefemengen zur effektiven Protektion bei Virusinfektionen führt.
3. Die Genexpression ist induzierbar und kann über Erhöhung der Konzentration des Transkriptionsaktivators Gal4 und/oder durch Codon-Optimierung der Nukleotidsequenz des Fremdgens in Anpassung an den Hefewirt weiter gesteigert werden. Die Etablierung eines Fed-Batch Fermentationsprotokolls erlaubt die effiziente Produktion auch cytotoxischer Antigene.
4. Die Integration des Fremdgens in *VAK367-D4* und Abkömmlingen hiervon ist eine "Ein-Schritt-Prozedur". D.h. in ca. 3 Wochen können neue rekombinante Stämme erzeugt werden; dies ist für die schnelle Entwicklung effizienter Impfstoffe gegen veränderte Virusvarianten besonders wichtig.
5. Durch subkutane Verabreichung rekombinanter Hefe des Typs *K*. *lactis,* speziell rekombinanter Hefe des Stammes *VAK367-D4* und Abkömmlingen hiervon, konnte sowohl in der Maus, als auch im Huhn eine protektive Immunantwort erzeugt werden. Die Prozedur ist denkbar einfach: Eine definierte Quantität inaktivierter (Hitze-abgetöteter) Hefezellen werden dem Impfling in einem 2-3 maligen Verfahren unter die Haut gespritzt. Zwei Wochen nach der letzten Anwendung wird das Serum des Impflings auf die Präsenz und Funktionalität antigen-spezifischer Antikörper hin untersucht. Durch Virusneutralisationstests konnte nachgewiesen werden, dass diese Immunantwort vorwiegend, wenn nicht ausschließlich auf der Erzeugung neutralisierender Antikörper beruhte (protektive humorale Immunantwort). Damit unterscheidet sich die durch *K. lactis* in der subkutanen Anwendung induzierbare Immunantwort grundlegend von der durch *S*. *cerevisiae* induzierbaren Immunantwort, die vor allem eine T-Zell Antwort induziert. Die Möglichkeiten einer subkutanen Anwendung von *K. lactis* sind daher grundsätzlich andere als die Möglichkeiten einer subkutanen Anwendung von *S. cerevisiae:* während *K. lactis* als *subunit* Vakzin bei Antigenen zur Anwendung kommen kann, die eine protektive humorale Immunantwort erzeugen können (z.B. virale Antigene wie das VP2 Antigen des Virus der infektiösen Bursitis, IBDV oder Hämagglutinin HA Antigen des Influenzavirus), so kann *S*. *cerevisiae* als *subunit* Vakzin bei Antigenen zur Anwendung kommen, die eine protektive zelluläre Immunantwort erzeugen können (wie z.B. beim NS3 Protein des Hepatitis C Virus oder Tumorantigenen wie dem Her-2). Zurückzuführen sind diese Unterschiede in der Form der induzierten Immunantwort vermutlich auf die stark unterschiedlichen Eigenschaften der *S*. *cervisiae* und *K*. *lactis* Zellen, die oben ausgeführt wurden

Zusammengefasst leistet die vorliegende Erfindung einen umfangreichen Beitrag zum Stand der Technik und stellt zahlreiche vorteilhafte Ausführungsformen gegenüber dem Stand der Technik bereit:
- Den Erfindern ist es gelungen, *subunit* Markervakzine herzustellen, mit denen es möglich ist, vakzinierte gegenüber natürlich infizierten Individuen zu unterscheiden.
- Weiterhin können *subunit* Markervakzine hergestellt werden, die zugleich stark adjuvierende Eigenschaften aufweisen und damit stark immunogen sind.
- Die erfindungsgemäßen *subunit* Markervakzine können mehrfach angewandt werden.
- Die erfindungsgemäßen *subunit* Markervakzine erzeugen eine systemische protektive Immunantwort und immunologisches *memory* im Impfling.
- Mit der vorliegenden Erfindung ist es auch möglich, Vakzine gegen cytotoxische Antigene herzustellen.
- Das erfindungsgemäße Verfahren erlaubt die möglichst schnelle Erzeugung neuer Vakzinvarianten.
- Die Vakzinierungsverfahren sind insbesondere sehr kostengünstig.
- Für die Herstellung der erfindungsgemäßen Vakzine sind keine Versuchstiere oder die Verwendung animaler oder humaner Zellen in Kultur notwendig.
- Die erfindungsgemäßen Vakzine sind nicht temperaturempfindlich, sie können ohne Kühlung transportiert und gelagert werden.
- In dem erfindungsgemäßen Verfahren werden keine lebenden rekombinanten Zellen oder Organismen verwendet.
- Mit dem erfindungsgemäßen Verfahren ist es möglich, sowohl die Quantitäten an verwendetem Vakzin, als auch die Anzahl an Anwendungen, die für das Erzielen einer protektiven Immunisierung notwendig sind, auf ein minimales Niveau zu beschränken.

### Ausführungsbeispiele

*1. Erzeugung des K. lactis Stammes VAK367-D4 (metA ura3-5 lac4::ScURA3).*
   Der Ausgangsstamm *VAK367* zur heterologen Expression von Fremdproteinen hat folgende Eigenschaften: Er erlaubt die Kultivierung zu hoher Zelldichte, ohne dass dabei intrazelluläre Proteine nachweisbar freigesetzt werden. Diesbezüglich unterscheidet sich dieser Stamm von vielen nahe verwandten *K. lactis* Stämmen. Der Stamm *VAK367* wurde durch zwei Mutagenese-Runden von dem Stamm CBS 2359 (Centraalbureau voor Schimmelcultures http://www.fungalbiodiversitycentre.com) abgeleitet und ist auxotroph für die Aminosäure Methionin und die Nucleobase Uracil. Vom Stamm *VAK367* wurde mit gentechnischen Methoden der Stamm *VAK367-D4* (hinterlegt am 18.11.2009 bei der Deutschen Sammlung von Mikroorganismen und Zellkulturen GmbH (DSMZ) in Braunschweig unter der Hinterlegungsnummer DSM 23097) abgeleitet, in dem mit Hilfe des Plasmids pD4-2 die Sequenz von +358 bis +1181 des *LAC4* Gens durch das *ScURA3* Gen ersetzt wurde. Der Stamm *VAK367-D4* erlaubt nun die Integration von Fremdgenen am *LAC4* Locus ohne zusätzliche Marker, indem auf Lactose-Wachstum selektiert wird. Dabei wird bei Benutzung eines geeigneten Integrationsvektors, wie z.B. Klp3-MCS **(****Abb. 6****)** durch homologe Rekombination die Disruptionskassette so ersetzt, dass unter Verlust des *ScURA3* Markers ein intaktes *LAC4* Gen rekonstituiert wird. **(****Abb.** 1)
*2. Erzeugung eines Integrationsvektors, der die induzierbare Expression von Fremdgenen erlaubt.*
   Vektor: Klp3
   Vektor: Klp3-MCS (SEQ ID Nr.: 10)
   Bei dem Vektor Klp3-MCS **(SEQ ID Nr.: 10) (****Abb. 6****)** handelt es sich um einen *E. coli* Vektor, basierend auf YRp7, der in Hefen nicht autonom replizieren kann, da die ARS1 Sequenz deletiert wurde. Klp3-MCS **(SEQ ID Nr.: 10)** enthält den *K.lactis LAC4* Promotor und Sequenzen, die die Integration am *LAC4* Locus durch homologe Rekombination ermöglichen.
   Zwischen *LAC4* Promotor und Transkriptionsstart wurde ein DNA Abschnitt inseriert, der den *TEF1* Terminator und den *KIGAL80* Promotor enthält. Dadurch kann der *LAC4* Leserahmen nach Rekonstitution über homologe Rekombination unter der Kontrolle des *KIGAL80* Promotors exprimiert werden. Der *KIGAL80* Promotor wird über den Transkriptionsfaktor *KlGal4* mit dem *LAC4* Promotor ko-reguliert (Zenke et al. 1993). Diese Konstruktion ermöglicht es, über Messung der *LAC4* codierten β-Galactosidase, die Induktion der Fremdgenexpression zu verfolgen. Klp3-MCS **(SEQ ID Nr.: 10**) erlaubt die Insertion des Fremdgens zwischen *LAC4* Promotor und *TEF1* Terminator über eine der unikalen Schnittstellen in der *multiple cloning site* (MCS) **(****Abb. 6****).** Zur Integration wird das resultierende Plasmid mit geeigneten Restriktionsenzymen verdaut, sodass die Expressionskassette von den *E. coli* Vektorsequenzen getrennt wird. Nach Transformation in *K. lactis VAK367-D4* wird die Expressionskassette chromosomal integriert; die resultierenden Stämme enthalten keine bakteriellen Sequenzen.
*3. K. lactis Variante, die das VP2-Antigen des Virus der infektiösen Bursitis (IBDV Variante D78) exprimiert.*
   *Herstellung des rekombinanten Hefestammes*
   Die cDNA, die für IBDV D78 VP2 kodiert, wurde aus dem Plasmid pD78A (Icard et al., 2008) mit Hilfe der folgenden Oligonukleotide amplifiziert:
   IBDV_AscI_fwd (5'-GGCGCGCCGATGACAAACCTGCAAGATC-3') **(SEQ ID NR.: 7),** enthaltend eine *AscI* Restriktionschnittstelle, und
   VP2_NotI_rev (5'-ATAAGAATGCGGCCGCTCACACAGCTATCCTCCTTATG-3') **(SEQ ID NR.: 8)** enthaltend eine *NotI* Restriktionsschnittstelle.
   Für die Generierung von VP2-T2S wurde folgendes Oligonukleotidpaar verwendet:
   IBDV_S:T_AscI_fwd (5'-GGCGCGCCGATGTCTAACCTGCAAGATCAAACCCA-3') **(SEQ ID NR.: 9)**, und VP2_NotI_rev (s.o.).

Die so amplifizierten DNA Fragmente wurden nach Überprüfung und Bestätigung der Nukleotidsequenzen über die *AscI* und *NotI* Schnittstellen in den Vektor Klp3-MCS **(SEQ ID Nr.: 10) (****Abb. 6****)** kloniert. Danach erfolgte die Integration in das Genom **(****Abb. 1****).** Im Einzelnen wurde das Integrationsplasmid mit dem Restriktionsenzym *EcoRI* verdaut und die verdauten Fragmente in kompetente *VAK367-D4* Zellen transformiert. Die transformierten Zellen wurden auf YEPD Medium plattiert und über Nacht bei 30°C inkubiert. Für das Auffinden von positiven Kolonien wurde die Transformationsplatte auf SM Medium, das Laktose als Kohlenstoffquelle enthielt, dupliziert und für 2 Tage bei 30°C inkubiert. Die bei diesem Verfahren identifizierten positiven Klone wurden weiter untersucht.

Die genomische Integration von zusätzlichen *KIGAL4* Genkopien wurde nach einer konventionellen Methode durchgeführt (Kuger et al. (1990). Die Codon-Optimierung folgte einem *Saccharomyces cerevisiae* Algorithmus (mr.gene.com, Raab et al., 2010). Die Codon-optimierten DNA Fragmente wurden direkt synthetisiert. Bei der Synthese wurden die 5' *AscI* und 3' *NotI* Restriktionsschnittstellen bereits eingebaut (mr.gene.com, Regensburg, Deutschland). Anschließend erfolgte die Klonierung in den Vektor Klp3-MCS (SEQ ID Nr.: 10).

### Western Blot Analyse.

Zellpellets wurden in B60 Puffer (50 mM HEPES-KOH pH 7.3; 60 mM Kaliumacetat; 5 mM Magnesiumacetat; 0,1 % Triton X100; 10 % Glycerol; 1 mM Natriumfluorid; 20 mM Glycerolphosphat; 10 mM MgCl₂; 1 mM DTT; Protease Komplettinhibitor (Roche)) resuspendiert und durch kräftiges Mixen mit Glasperlen aufgeschlossen. Der Extrakt wurde zentrifugiert (14000 U/min, 20 min bei 4°C) und die Proteinkonzentration bestimmt. 40 µg des Proteinextraktes wurden mittels SDS-PAGE in einem 12% Gel getrennt. Danach wurden die Proteine auf eine Membran übertragen. Die Western Blot Analysen wurden mit einem α-IBDV Antiserum aus Kaninchen (1:15,000; Granzow et al., 1997) und einem Ziegen-α-Kaninchen HRPgekoppelten Antikörper (1:3000, Santa Cruz Biotechnology, Inc.) unter Nutzung konventioneller Methoden durchgeführt.

### Northern Blot Analyse.

Für die vollständige Extraktion der RNA wurden 5 ml einer Hefekultur auf Eis gekühlt. Die Zelllyse wurde in Prot K Puffer (100 mM Tris/ HCl pH 7.9, 150 mM NaCl, 25 mM EDTA, 1 % SDS) und 50 mg Proteinase K (Fermentas) unter starkem Schütteln mit Glasperlen durchgeführt. Die Proben wurden 1 h bei 35 °C inkubiert und die RNA extrahiert, mit Ethanol gefällt und in DEPC Wasser resuspendiert. Die Northern-Analyse wurde wie in Engler-Blum et al., 1993 beschrieben, allerdings mit einigen Abweichungen, durchgeführt. 5 µg der totalen RNA wurden auf einem 1 % Formaldehyd-Agarosegel getrennt und auf eine Nylon Membran (Amersham HybondTM-N+, GE Healthcare) übertragen. Die Membran wurde bei 68°C mit einer DIG-markierten RNA Sonde inkubiert, die durch *in vitro* Transkription von PCR Fragmenten in Anwesenheit von DIG-NTPs (Roche) hergestellt wurde. Der Blot wurde mit einer Blockierungslöung behandelt und mit einem anti-DIG alkalische-Phosphatase-konjugiertem Antikörper (Roche) inkubiert. Die Bestimmung der Aktivität der alkalischen Phosphatase wurde mittels konventioneller Methoden durchgeführt.

### Quantifizierung von heterolog exprimierten VP2.

Verwendet wurde ein modifiziertes Protokoll nach Saugar et al., 2005. 2000 ODE einer Hefekultur, die mit einem episomalen VP2 Plasmid (pADH1-P VP2-T2S) transformiert war, wurden auf selektivem Medium (0,67% YNB, 2% Glukose und folgende Zusätze: 11 mg/l Ade; 14 mg/l Tyr; je 38 mg/l His, Trp, Arg, Met; 48 mg/l Phe; je 58 mg/l Leu, Iie, Lys, Val, Thr) über Nacht kultiviert. Nach Ernte und Waschen mit destilliertem Wasser wurden die Zellen mit Glasperlen in Lysispuffer (10 mM Tris (pH 8.0), 150 mM NaCl, 20 mM CaCl₂, 1 mM EDTA, Protease Komplettinhibitor (Roche), pH 8.0) aufgeschlossen. Der resultierende Proteinextrakt wurde zentrifugiert (10,000g für 1h bei 4°C) und die lösliche Fraktion auf ein 20% (w/v) Sucrosekissen in Sucrosepuffer geschichtet (10mM Tris pH 8.0, 150 mM NaCl, 20 mM CaCl₂; enthielt Protease Komplettinhibitor (Roche)). Nach Zentrifugation bei 170,000 g für 3 h bei 4°C wurde das Pellet in 200 µl Sucrosepuffer gelöst und weitere 17 h bei 114.000 g in einem 20 bis 53 % Sucrosegradienten in Sucrosepuffer zentrifugiert. Der Gradient wurde in 700 µl Fraktionen aufgefangen und mittels SDS-PAGE und Western Blot analysiert. Oligomere Proteinkomplexe des heterolog exprimierten VP2 konnten auf diese Weise konzentriert und aufgereinigt werden. Das Protein konnte nachgewiesen und die Proteinmenge mittels SDS PAGE und Coomassie Färbung im Vergleich zu einem Standardprotein bestimmt werden (nicht gezeigt). Das so gereinigte VP2 wurde dann als Standard in einem vergleichenden Westernblot mit anti-VP2 Antikörper eingesetzt. Verglichen wurde die VP2 Menge einer definierten Zahl von Hefezellen aus verschiedenen Fermentationen **(****Abb. 3****).**

### Hefefermentation und Hitzeinaktivierung.

Alle experimentellen Fermentationen wurden in einem DasGip Parallelbioreaktorsystem (DasGip AG, Jülich, Deutschland) mit vier voll ausgerüsteten 2I Fermentoren durchgeführt. Fermentationen im Produktionsmaßstab wurden durch die Firma Organobalance GmbH (Berlin, Deutschland) oder im eigenen Labor in einem Biostat ED Bioreactor (B. Braun Biotech, Melsungen, Deutschland) mit 10I Arbeitsvolumen durchgeführt. Alle Produktionsprozesse wurden im Fed-Batch Verfahren durchgeführt. Es wurde ein komplexes Kulturmedium mit 2% Hefe-Extrakt und 1% Pepton und eine 20%ige Lactose-Feed-Lösung benutzt. Die Temperatur der Hefekultur wurde bei 30°C gehalten und der pO₂ wurde auf 30% Sättigung gesteuert. Der pH-Wert wurde während der Fermentation auf 5.0 durch Zugabe von 2M NaOH oder 2M H₃PO₄ gehalten.

Für die *in vivo* Experimente in Mäusen und Hühnern wurden die Hefen gefriergetrocknet und danach für 2 h bei 90°C hitzeinaktiviert. Nach Nutzung dieses Verfahrens waren weniger als 10 Zellen pro Gramm Zelltrockengewicht lebensfähig.

### 4. Subkutane Applikation in Mäusen

Für die subkutane Applikation einer *K*. *lactis* Variante, die das VP2-Antigen des Virus der infektiösen Bursitis (IBDV Variante D78) exprimiert (VAK890), in Mäusen wurde die getrocknete und pulverisierte Hefe für die erste Anwendung mit komplettem Freund-Adjuvanz (CFA) gemischt; in den weiteren Anwendungen wurde die Hefe mit inkompletten Freund-Adjuvanz (IFA) gemischt (100 µg Hefematerial pro 200 µl CFA oder IFA). 200 µl der Emulsionen (mit enthaltenen 100 µg Hefe) wurde pro Immunisierung/boost pro Individuum injiziert. Damit entsprach die pro subkutane Immunisierung an ein Mausindividuum verabreichte Menge an VP2 ca. 18 ng **(****Abb. 3****)** Nach der initialen Injektion (Tag 0) wurde zweimal in zwei Wochen-Intervallen "geboosted" (an Tag 14 und 28; **Abb. 4**). Nach weiteren zwei Wochen wurden die Tiere zur Gewinnung des Blutserums durch Narkose getötet.

### 5. Subkutane Applikation in Hühnern

Für die subkutane Applikation in Hühnern wurden 5 mg der getrockneten und pulverisierten *K. lactis* Variante, die das VP2-Antigen des Virus der infektiösen Bursitis (IBDV Variante D78) exprimiert (VAK890), in 750 µl Phosphatpuffer/Saline (PBS) sowie 500 µl sterile destilliertem Wasser gelöst und eine Emulsion mit 1.25 ml IFA hergestellt. 500 µl dieser Emulsion (mit enthaltenen 1 mg Hefe) wurden an Tag 0, 14 sowie Tag 28 injiziert **(****Abb. 5****).** Damit entsprach die pro subkutane Immunisierung eines Huhnindividuums verabreichte Menge an VP2 ca. 180 ng **(****Abb. 3****,** **4**).

### 6. Virus "challenge"

Nach der Vakzinierung **(****Abb. 5****)** wurden Huhnimpflinge an Tag 42 über den oralen Weg mit 100 EID₅₀ des IBDV Stammes "Edgar" infiziert und nach sechs Tagen die Mortalitätsrate bestimmt. Nach anschließender Tötung der Tiere unter Narkose wurden die Seren gewonnen und die Bursae der Tiere entnommen. Diese wurden zunächst für 24 Stunden in 10% neutral gepuffertem Formalin fixiert und anschließend in Paraffin eingebettet.

### 7. Enzyme-linked immunosorbent assay (ELISA).

Die IBDV spezifischen Antikörpertiter in den Seren der Impflinge wurden über einen käuflichen ELISA-Test bestimmt IDEXX FlockChek® IBD ELISA kit (IDEXX Laboratories, Inc.). Im Falle der Seren aus Mausimpflingen wurde ein vom Hersteller abweichender sekundärer Antikörper verwendet (Sigma Aldrich).

### 8. Neutralisations-Assay.

Der Neutralisationsassay zur Bestimmung der Konzentration Virus-neutralisierender Antikörper wurde nach dem Protokoll von Schröder et al., 2000 durchgeführt.

### 9. Immunhistochemie.

Aus den in Paraffin eingebetteten Bursae wurden 4 Mikrometer dicke Organschnitte hergestellt. Nach Entfernung des Paraffins wurden diese nach Standardprozeduren mit Haematoxylin und Eosin angefärbt. Die Proben wurden mikroskopisch untersucht und der sog. "Läsions score" in einer Skala von 1-4 bestimmt (1 = normal bis 10% follikuläre Atrophie; 2 = 10-30% follikuläre Atrophie; 3 = 30-70% follikuläre Atrophie; 4 = > 70% Atrophie).

### Ergebnisse

### Herstellung und Optimierung des IBDV VP2 exprimierenden K. lactis Stammes

Es wurden verschiedene *K. lactis* Varianten mit integriertem IBDV VP2 Gen hergestellt. Für die Vakzinierungsexperimente wurde eine optimierte Variante verwendet, bei der das VP2 Protein an Aminosäureposition 2 mutiert war (Austausch Threonin gegen Serin; Jagadish et al. (1991)), und die eine zusätzliche tandem-Integration von mindestens zwei *KIGAL4* Genen enthielt (Variante VP2-T2S_GAL4; Stamm VAK890). Durch die Mutation wurde das Fremdprotein zusätzlich stabilisiert; durch die Überexpression des Trans-Aktivators konnte eine deutliche Steigerung der VP2 Expression erreicht werden **(****Abb. 2****).** Die Integration zusätzlicher *KIGAL4* Gene korrelierte auch mit einer höheren Wachstumsrate dieser *K*. *lactis* Variante. Die Wachtumsbedingungen für den betreffenden VP2-exprimierenden *K*. *lactis* Stamm VAK890 wurden optimiert, so dass sich die Hefe in hohen Dichten und mit reproduzierbarer Quantität an exprimiertem VP2 fermentieren ließ. Nach der Herstellung wurde die Hefe gefriergetrocknet und bei 90° C für 2 Stunden inaktiviert. Der Nachweis der Inaktivierung wurde geführt: Pro g inaktiviertem Hefematerial verblieben weniger als 10 lebende Hefezellen. Die Quantität an VP2 pro Hefezelle wurde bestimmt: Sie betrug mit dem Stamm VAK890 ca. 0.7 fg heterologes VP2 Protein pro Hefezelle **(****Abb. 3****)**

### Subkutane Applikation in Mäusen und Hühnern

Die Immunisierungen wurden wie oben beschrieben durchgeführt; zwei Wochen nach der letzten Applikation wurden die Sera der behandelten Impflinge auf die Präsenz neutralisierender Antikörper hin untersucht. Dazu wurde ein IBDV-spezifischer ELISA verwendet und ein IBDV Neutralisationsassay durchgeführt **(****Abb. 4** **und** **5****).** Mit den vakzinierten Hühnern wurde zudem ein "*Virus-challenge*" Experiment durchgeführt. Dazu wurde den Tieren eine Virusdosis von 100 EID50 pro Tier des stark virulenten IBDV Stamms "Edgar" zugeführt, eine Konzentration, die bei nicht vakziniertem Geflügel zu einer signifikanten Bursitis mit einer Mortalitätsrate von ca. 10-35% **(****Abb. 5D****)** führt. Im Anschluss an das "Viruschallenge" Experiment wurden die Bursae der Impflinge über Immunhistochemie auf Infektionsanzeichen und Läsionen in den Bursae hin untersucht und durch den sog. "Läsionen score" **(****Abb. 5****)** charakterisiert. Sowohl die Experimente mit Mäusen, als auch die Experimente mit Hühnern zeigten, dass durch die subkutane Applikation der *K*. *lactis* Stamm VAK890 hohe Titer Virus-neutralisierender Antikörper in praktisch allen behandelten Tieren erzeugt werden konnten **(****Abb. 4B****,** **4C****;** **Abb. 5B****,** **5C****).** Ebenso konnte gezeigt werden, dass praktisch alle vakzinierten Huhnprobanden gegen Viruschallenge geschützt waren und praktisch keinerlei Zeichen einer Virusinfektion in ihren Bursae aufwiesen **(****Abb. 5****).** Alle subkutan mit dem *K. lactis* Stamm VAK890 inokulierten Tiere zeigten somit eine signifikante humorale Immunantwort gegen VP2. Diese Immunantwort war bereits nach einem einmaligen boost zu beobachten, woraus zu schließen ist, dass zwei Injektionen, die zudem mit inkomplettem Freund Adjuvanz durchgeführt werden können (Immunisierung und ein boost), bereits ausreichend sind, um einen Schutz herzustellen Zudem waren alle Huhnprobanden, die mit dem *K*. *lactis* Stamm VAK890 inokuliert waren, gegen eine nachfolgende Virusinfektion geschützt **(****Abb. 5****).**

### Abkürzungen

- ARS1: autonom replizierende Sequenz; Nukleotidsequenz auf der DNA, an dem die Replikation eingeleitet wird
- Asc I: Restriktionsendonuklease Asc I
- CFA: komplettes Freund-Adjuvanz
- DNA: Deoxribonucleic acid
- DEPC: Diethylpyrocarbonat
- DIG-NTP: Digoxigenin-Nukleotidtriphosphat
- DSMZ: Deutsche Sammlung von Mikroorganismen und Zellkulturen GmbH
- DTT: Dithiothreithol
- E. coli: *Escherichia coli*
- EcoRI: Restriktionsendonuklease EcoR I
- EDTA: Ethylendiamintetraessigsäure
- EID50: Egg oder Embryo Infectious dose - Anzahl an infektiösen Viren, die notwendig ist, um bei 50% infizierter Eier eine Infektion auszulösen
- ELISA: Enzyme-linked immunosorbent assay
- GAL4: hefespezifischer Transkriptionsaktivator
- GRAS: generally regarded as safe
- HEPES: 2-(4-(2-Hydroxyethyl)- 1-piperazinyl)-ethansulfonsäure
- Hpa I: Restriktionsendonuklease Hpa I
- HRP: horseradish peroxidase
- IBDV: Virus der Infektiösen Bursitis
- IFA: inkomplettes Freund-Adjuvanz
- *K. lactis*: *Kluyveromyces lactis*
- *KlGAL4*: *K. lactis* Gen codierend für das KlGal4/Lac9 Protein
- *KIGAL80*: *K. lactis* Gen codierend für das KlGal80 Protein
- *LAC4*: *K. lactis* Gen codierend für ein β-Galactosidase-Enzym
- Not I: Restriktionsendonuklease Not I
- ODE: Optische Dichte Einheit
- PBS: Phosphatpuffer/Saline
- PCR: Polymerase chain reaction
- RNA: Ribonucleic acid
- *S*. *cerevisiae*: *Saccharomyces cerevisiae*
- *Sal* I: Restriktionsendonuklease Sal I
- SDS: Sodiumdodekylsulfat
- SDS-PAGE: Polyacrylamidgelelekrophorese unter Verwendung von SDS
- TEF1: *Arxula adeninivorans* Gen codierend für den Translationsfaktor EF-1 alpha
- VP2: Capsidbildendes Virusprotein des IBDV
- VP2-T2S: VP2 mit einem Aminosäureaustausch Threonin gegen Serin an Position 2
- VAK: Vakzinstamm
- YEPD: Yeast Extract Peptone Dextrose
- YRp7: *S*. *cerevisiae-E.coli shuttle* Vektor, Genbank Accession U03501 (Botstein et al., 1979)

### Referenzliste

Backhaus, K. et al. Milk and sugar: Regulation of cell wall synthesis in the milk yeast Kluyveromyces lactis. European Journal of Cell Biology 90, 745-750 (2011).
Bathurst, I.C. Protein Expression in Yeast as an Approach to Production of Recombinant Malaria Antigens. The American Journal of Tropical Medicine and Hygiene 50, 20 -26 (1994).
Botstein D, Falco, S.C., Stewart, S.E., Brennan, M., Scherer, S., Stinchcomb, D.T., Struhl, K. & Davis, R.W. Sterile host yeast (SHY): a eukaryotic system of biological containment for recombinant DNA experiments. Gene 8, 17-24 (1979).
Breunig et al. Regulation of primary carbon metabolism in Kluyveromyces lactis. Enzyme Microb. Technol 26, 771-780 (2000).
Chen, X.J. & Clark-Walker, G.D. Specific mutations in alpha- and gamma-subunits of F1-ATPase affect mitochondrial genome integrity in the petite-negative yeast Kluyveromyces lactis. EMBO J 14, 3277-3286 (1995).
Clark-Walker, G.D. The F1-ATPase inhibitor Inh1 (IF1) affects suppression of mtDNA loss-lethality in Kluyveromyces lactis. FEMS Yeast Research 7, 665-674 (2007).
Donnini, C. et al. Improved Production of Heterologous Proteins by a Glucose Repression-Defective Mutant of Kluyveromyces lactis. Appl Environ Microbiol 70, 2632-2638 (2004).
Engler-Blum, G., Meier, M., Frank, J. & Muller, G.A. Reduction of Background Problems in Nonradioactive Northern and Southern Blot Analyses Enables Higher Sensitivity Than 32P-Based Hybridizations. Analytical Biochemistry 210, 235-244 (1993).
Gellissen G., Hollenberg C.P. Application of yeasts in gene expression studies: a comparison of Saccharomyces cerevisiae, Hansenula polymorpha and Kluyveromyces lactis -- a review. Gene 190(1), 87-97 (1997).
Granzow, H. et al. A second form of infectious bursal disease virus-associated tubule contains VP4. J Virol 71, 8879-8885 (1997).
Jagadish, M.N., Laughton, D.L., Azad, A.A. & Macreadie, I.G. Stable synthesis of viral protein 2 of infectious bursal disease virus in Saccharomyces cerevisiae. Gene 108, 275-279 (1991).
Icard, A.H., Sellers, H.S., & Mundt, E. Detection of infectious bursal disease virus isolates with unknown antigenic properties by reverse genetics. Avian Dis. 52, 590-8. (2008)
Kuger, P., Gödecke, A. & Breunig, K.D. A mutation in the Zn-finger of the GAL4 homolog LAC9 results in glucose repression of its target genes. Nucleic Acids Res 18, 745-751 (1990).
Lu, Y. et al. Mutation-Selective Tumor Remission with Ras-Targeted, Whole Yeast-Based Immunotherapy. Cancer Research 64, 5084 -5088 (2004).
Raab, D., Graf, M., Notka, F., Schödl, T. & Wagner, R. The GeneOptimizer Algorithm: using a sliding window approach to cope with the vast sequence space in multiparameter DNA sequence optimization. Syst Synth Biol 4, 215-225 (2010).
Raschke, W.C. & Ballou, C.E. Characterization of a yeast mannan containing N-acetyl-D-glucosamine as an immunochemical determinant. Biochemistry 11, 3807-3816 (1972).
Saugar, I. et al. Structural Polymorphism of the Major Capsid Protein of a Double-Stranded RNA Virus: An Amphipathic [alpha] Helix as a Molecular Switch. Structure 13, 1007-1017 (2005).
Schröder, A., van Loon, A.A.W.M., Goovaerts, D. & Mundt, E. Chimeras in noncoding regions between serotypes I and II of segment A of infectious bursal disease virus are viable and show pathogenic phenotype in chickens. Journal of General Virology 81, 533 -540 (2000).
Stubbs, A.C. et al. Whole recombinant yeast vaccine activates dendritic cells and elicits protective cell-mediated immunity. Nat. Med 7, 625-629 (2001).
Stubbs, A.C. and Wilson, C.C. Recombinant yeast as a vaccine vector for the induction of cytotoxic T-lymphocyte responses. Curr. Opin. Mol. Ther. 4: 35-40 (2002)
Uccelletti, D., Farina, F., Mancini, P. & Palleschi, C. KIPMR1 inactivation and calcium addition enhance secretion of non-hyperglycosylated heterologous proteins in Kluyveromyces lactis. Journal of Biotechnology 109, 93-101 (2004).
Van Ooyen, A.J.J. et al. Heterologous protein production in the yeast Kluyveromyces lactis. FEMS Yeast Research 6, 381-392 (2006).
Wansley E.K. et al., Vaccination with a recombinant Saccharomyces cerevisiae expressing a tumor antigen breaks immune tolerance and elicits therapeutic antitumor responses. Clin. Cancer Res. 14: 4316-4325 (2008).
Zenke et al. Gal80 proteins of Kluyveromyces lactis and Saccharomyces cerevisiae are highly conserved but contribute differently to glucose repression of the galactose regulon. Molecular and Cellular Biology 13:7566-7576 (1993)

## Patentansprüche

1. Rekombinante Hefe der Spezies *Kluyveromyces lactis,* die ein Gen, das für ein VP2-Antigen des Virus der Infektiösen Bursitis (IBDV) codiert, als Fremdgen trägt, das in das Hefegenom integriert ist, und die die Expression des VP2-Antigens des Virus der Infektiösen Bursitis (IBDV) als Fremdprotein ermöglicht, **dadurch gekennzeichnet, dass** dieser *Kluyveromyces lactis* Stamm ausgewählt ist aus:
*Kluyveromyces lactis* DSM 25405,
*Kluyveromyces lactis* DSM 25406, und
*Kluyveromyces lactis* DSM 25407.

2. Rekombinante Hefe nach Anspruch 1, **dadurch gekennzeichnet, dass** die Fremdgenexpression konstitutiv erfolgt oder dass die Fremdgenexpression induzierbar ist.

3. Rekombinante Hefe nach Anspruch 1 oder 2, **dadurch gekennzeichnet, dass** die Fremdgenexpression indirekt über die Expression eines endogenen Reportergens quantifiziert werden kann.

4. Rekombinante Hefe nach einem oder mehreren der vorherigen Ansprüche zur Verwendung in einem Verfahren zur subkutanen Vakzinierung.

5. Rekombinante Hefe nach Anspruch 4, **dadurch gekennzeichnet, dass** die rekombinanten Hefen als *subunit* Markervakzine verwendet werden.

6. Rekombinante Hefe nach Anspruch 5, **dadurch gekennzeichnet, dass** die *subunit* Markervakzine dafür verwendet werden, vakzinierte gegenüber natürlich infizierten Individuen zu unterscheiden.

7. Rekombinante Hefe nach Anspruch 5 oder 6, **dadurch gekennzeichnet, dass** die *subunit* Markervakzine zugleich stark adjuvierende Eigenschaften aufweisen.

8. Rekombinante Hefe nach einem der Ansprüche 5 bis 7, **dadurch gekennzeichnet, dass** die *subunit* Markervakzine stark immunogen sind.

9. Rekombinante Hefe nach Anspruch 1 zur Verwendung in einem Verfahren zur subkutanen Vakzinierung mittels kompletter Hefezellen einer rekombinanten Hefe, **dadurch gekennzeichnet, dass** eine protektive humorale Immunisierung gegen exprimiertes Fremdprotein erzeugt wird und das Verfahren die folgenden Schritte umfasst:
a) Anzucht und Vermehrung der rekombinanten Hefen,
b) Ernte und Inaktivierung der Hefen,
c) Applikation der rekombinanten Hefen gemäß einem festzulegenden Immunisierungsschema,
d) Titerbestimmung der gebildeten Antikörper und/oder
e) Nachweis der Immunisierung.

10. Rekombinante Hefe zur Verwendung nach Anspruch 9, **dadurch gekennzeichnet, dass** mittels subkutaner Applikation kompletter Hefezellen einer rekombinanten Hefe der Spezies *Kluyveromyces lactis* eine protektive humorale Immunisierung gegen exprimiertes Fremdprotein erzeugt wird.

11. Oligonukleotidpaar, das eine Nukleinsäuresequenz gemäß SEQ ID NR.: 8 und SEQ ID NR.: 9 aufweist.

12. Expressionsvektor Klp3 oder Klp3-MCS gemäß SEQ ID NR.: 10, der ein Fremdgen trägt, **dadurch gekennzeichnet, dass** das Fremdgen die Nukleinsäuresequenz gemäß SEQ ID NR.: 3 oder SEQ ID NR.: 5 aufweist, zur Integration in den *Kluyveromyces lactis* Ausgangsstamm VAK367-D4, hinterlegt unter DSM 23097.

13. Expressionsvektor nach Anspruch 12, **dadurch gekennzeichnet, dass** das Fremdgen für das IBDV VP2-T2S Protein mit der Aminosäuresequenz gemäß SEQ ID NR.: 4 oder das IBDV oVP2-T2S Protein mit der Aminosäuresequenz gemäß SEQ ID NR.: 6 kodiert.

## Claims

1. Recombinant yeast of the species *Kluyveromyces lactis* which carries as a foreign gene a gene that codes for a VP2 antigen of the virus of infectious bursitis (IBDV), which is integrated into the yeast genome, and which enables the expression of the VP2 antigen of the virus Infectious Bursitis (IBDV) as a foreign protein, **characterised in that** this *Kluyveromyces lactis* strain is selected from:
*Kluyveromyces lactis* DSM 25405,
*Kluyveromyces lactis* DSM 25406, and
*Kluyveromyces lactis* DSM 25407.

2. Recombinant yeast pursuant to Claim 1, **characterised in that** the foreign gene expression is constitutive or that the foreign gene expression is inducible.

3. Recombinant yeast pursuant to Claim 1 or 2, **characterised in that** the foreign gene expression can be quantified indirectly via the expression of an endogenous reporter gene.

4. Recombinant yeast pursuant to one or more of the preceding claims for use in a subcutaneous vaccination method.

5. Recombinant yeast pursuant to Claim 4, **characterised in that** the recombinant yeasts are used as *subunit* marker vaccines.

6. Recombinant yeast pursuant to Claim 5, **characterised in that** the *subunit* marker vaccines are used to distinguish between vaccinated and naturally infected individuals.

7. Recombinant yeast pursuant to Claim 5 or 6, **characterised in that** the *subunit* marker vaccines also have strong adjuvant properties.

8. Recombinant yeast pursuant to one of Claims 5 to 7, **characterised in that** the *subunit* marker vaccines are highly immunogenic.

9. Recombinant yeast pursuant to Claim 1 for use in a method for subcutaneous vaccination using complete yeast cells of a recombinant yeast, **characterised in that** a protective humoral immunisation against expressed foreign protein is produced, with the method comprising the following steps:
a) cultivation and propagation of the recombinant yeasts,
b) harvest and inactivation of the yeasts,
c) application of the recombinant yeasts pursuant to a defined immunisation scheme,
d) titre determination of the formed antibodies and/or
e) evidence of immunisation.

10. Recombinant yeast for use pursuant to Claim 9, **characterised in that**, by means of subcutaneous application of complete yeast cells of a recombinant yeast of the species *Kluyveromyces lactis,* a protective humoral immunisation against expressed foreign protein is produced.

11. Oligonucleotide pair having a nucleic acid sequence pursuant to SEQ ID NO.: 8 and SEQ ID NO.: 9.

12. Expression vector Klp3 or Klp3-MCS pursuant to SEQ ID NO.: 10, which carries a foreign gene, **characterised in that** the foreign gene has the nucleic acid sequence pursuant to SEQ ID NO.: 3 or SEQ ID NO.: 5, for integration in the *Kluyveromyces lactis* starting strain VAK367-D4, deposited under DSM 23097.

13. Expression vector pursuant to Claim 12, **characterised in that** the foreign gene for the IBDV VP2-T2S protein with the amino acid sequence pursuant to SEQ ID NO.: 4 or the IBDV oVP2-T2S protein encodes with the amino acid sequence pursuant to SEQ ID NO: 6.

## Revendications

1. Levure recombinante de l'espèce *Kluyveromyces lactis,* laquelle porte en tant que gène étranger un gène en charge de codifier un antigène VP2 du virus de la bursite infectieuse (IBD). Ce gène étranger est intégré dans le génome de levure et permet l'expression de l'antigène VP2 du virus de la bursite infectieuse (IBD) en qualité de protéine étrangère, **caractérisée en ce que** cette souche *Kluyveromyces lactis* est sélectionnée de :
*Kluyveromyces lactis* DSM 25405,
*Kluyveromyces lactis* DSM 25406, et
*Kluyveromyces lactis* DSM 25407.

2. Levure recombinante selon la revendication 1, **caractérisée en ce que** l'expression du gène étranger s'établit de manière constructive ou que cette dernière est inductible.

3. Levure recombinante selon la revendication 1 ou 2, **caractérisée en ce que** l'expression du gène étranger peut être quantifiée indirectement par l'expression d'un gène rapporteur endogène.

4. Levure recombinante selon une ou plusieurs des revendications précitées relatives à l'application dans une procédure de vaccination sous-cutanée.

5. Levure recombinante selon la revendication 4, **caractérisée en ce que** les levures recombinantes sont utilisées en qualité de vaccins marqueurs *subunit.*

6. Levure recombinante selon la revendication 5, **caractérisée en ce que** les vaccins marqueurs *subunit* sont utilisés afin de différencier les individus vaccinés des individus naturellement contaminés.

7. Levure recombinante selon la revendication 5 ou 6, **caractérisée en ce que** les vaccins marqueurs *subunit* présentent également des propriétés adjuvantes.

8. Levure recombinante selon une des revendications 5 à 7, **caractérisée en ce que** les vaccins marqueurs *subunit* sont fortement immunogènes.

9. Levure recombinante selon la revendication 1 relative à l'application dans une procédure de vaccination sous-cutanée au moyen de cellules de levure complètes d'une levure recombinante, **caractérisée en ce qu'**une immunisation humorale protectrice est générée contre la protéine étrangère exprimée et **en ce que** la procédure intègre les étapes suivantes :
a) Culture et prolifération des levures recombinantes,
b) Récolte et inactivation des levures,
c) Application des levures recombinantes conformément à un schéma d'immunisation à définir,
d) Titration des anticorps formés et/ou
e) Démonstration de l'immunisation.

10. Levure recombinante pour l'application relatée selon la revendication 9, **caractérisée en ce qu'**une immunisation humorale protectrice est générée contre la protéine étrangère exprimée au moyen d'une application sous-cutanée de cellules de levure complètes d'une levure recombinante de l'espèce *Kluyveromyces lactis.*

11. Paire d'oligonucléotides, présentant une séquence d'acide nucléique conforme à la mention SEQ ID NO : 8 et SEQ ID NO : 9.

12. Vecteur d'expression klp3 ou klp3-MCS conforme à la mention SEQ ID NO : 10, lequel porte un gène étranger, **caractérisé en ce que** ledit gène étranger présente la séquence d'acide nucléique conforme à la mention SEQ ID NO : 3 ou SEQ ID NO : 5, répertorié à des fins d'intégration dans *Kluyveromyces lactis,* souche initiale VAK367-D4, sous la désignation DSM 23097.

13. Vecteur d'expression selon la revendication 12, **caractérisé en ce que** le gène étranger codifie pour la protéine IBD VP2-T2S avec la séquence d'acide nucléique conforme à la mention SEQ ID NO : 4 ou la protéine IBD oVP2-T2S avec la séquence d'acide nucléique conforme à la mention SEQ ID NO : 6.


### SEQUENCE LISTING

<110> Martin-Luther-Universität Halle-Wittenberg
<120> Erzeugung einer humoralen Immunantwort mittels Hefe-basierter vakzinierung
<130> MLU_Breu_1
<160> 10
<170> BiSSAP 1.0
<210> 1
   <211> 1371
   <212> DNA
   <213> Birnaviridae
<220>
   <221> source
   <222> 1..1371
   <223> /mol_type="DNA" /organism="Birnaviridae"
<400> 1
   atgacaaacc tgcaagatca aacccaacag attgttccgt tcatacggag ccttctgatg      60
   ccaacaaccg gaccggcgtc cattccggac gacaccctgg agaagcacac tctcaggtca     120
   gagacctcga cctacaattt gactgtgggg gacacagggt cagggctaat tgtctttttc     180
   cctggattcc ctggctcaat tgtgggtgct cactacacac tgcagggcaa tgggaactac     240
   aagttcgatc agatgctcct gactgcccag aacctaccgg ccagttacaa ctactgcagg     300
   ctagtgagtc ggagtctcac agtgaggtca agcacacttc ctggtggcgt ttatgcacta     360
   aacggcacca taaacgccgt gaccttccaa ggaagcctga gtgaactgac agatgttagc     420
   tacaatgggt tgatgtctgc aacagccaac atcaacgaca aaattgggaa cgtcctagta     480
   ggggaagggg tcaccgtcct cagcttaccc acatcatatg atcttgggta tgtgaggctt     540
   ggtgacccca ttcccgcaat agggcttgac ccaaaaatgg tagccacatg tgacagcagt     600
   gacaggccca gagtctacac cataactgca gccgatgatt accaattctc atcacagtac     660
   caaccaggtg gggtaacaat cacactgttc tcagccaaca ttgatgccat cacaagcctc     720
   agcgttgggg gagagctcgt gtttcaaaca agcgtccacg gccttgtact gggcgccacc     780
   atctacctca taggctttga tgggacaacg gtaatcacca gggctgtggc cgcaaacaat     840
   gggctgacga ccggcaccga caaccttatg ccattcaatc ttgtgattcc aacaaacgag     900
   ataacccagc caatcacatc catcaaactg gagatagtga cctccaaaag tggtggtcag     960
   gcaggggatc agatgtcatg gtcggcaaga gggagcctag cagtgacgat ccatggtggc    1020
   aactatccag gggccctccg tcccgtcacg ctagtggcct acgaaagagt ggcaacagga    1080
   tccgtcgtta cggtcgctgg ggtgagcaac ttcgagctga tcccaaatcc tgaactagca    1140
   aagaacctgg ttacagaata cggccgattt gacccaggag ccatgaacta cacaaaattg    1200
   atactgagtg agagggaccg tcttggcatc aagaccgtct ggccaacaag ggagtacact    1260
   gactttcgtg aatacttcat ggaggtggcc gacctcaact ctcccctgaa gattgcagga    1320
   gcattcggct tcaaagacat aatccgggcc ataaggagga tagctgtgtg a             1371
<210> 2
   <211> 456
   <212> PRT
   <213> Birnaviridae
<220>
   <221> SOURCE
   <222> 1..456
   <223> /mol_type="protein" /organism="Birnaviridae"
<400> 2
   Met Thr Asn Leu Gln Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Gln Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg Ile Ala Val
       450                 455
<210> 3
   <211> 1371
   <212> DNA
   <213> Birnaviridae
<220>
   <221> source
   <222> 1..1371
   <223> /mol_type="DNA" /organism="Birnaviridae"
<400> 3
   atgtctaacc tgcaagatca aacccaacag attgttccgt tcatacggag ccttctgatg      60
   ccaacaaccg gaccggcgtc cattccggac gacaccctgg agaagcacac tctcaggtca     120
   gagacctcga cctacaattt gactgtgggg gacacagggt cagggctaat tgtctttttc     180
   cctggattcc ctggctcaat tgtgggtgct cactacacac tgcagggcaa tgggaactac     240
   aagttcgatc agatgctcct gactgcccag aacctaccgg ccagttacaa ctactgcagg     300
   ctagtgagtc ggagtctcac agtgaggtca agcacacttc ctggtggcgt ttatgcacta     360
   aacggcacca taaacgccgt gaccttccaa ggaagcctga gtgaactgac agatgttagc     420
   tacaatgggt tgatgtctgc aacagccaac atcaacgaca aaattgggaa cgtcctagta     480
   ggggaagggg tcaccgtcct cagcttaccc acatcatatg atcttgggta tgtgaggctt     540
   ggtgacccca ttcccgcaat agggcttgac ccaaaaatgg tagccacatg tgacagcagt     600
   gacaggccca gagtctacac cataactgca gccgatgatt accaattctc atcacagtac     660
   caaccaggtg gggtaacaat cacactgttc tcagccaaca ttgatgccat cacaagcctc     720
   agcgttgggg gagagctcgt gtttcaaaca agcgtccacg gccttgtact gggcgccacc     780
   atctacctca taggctttga tgggacaacg gtaatcacca gggctgtggc cgcaaacaat     840
   gggctgacga ccggcaccga caaccttatg ccattcaatc ttgtgattcc aacaaacgag     900
   ataacccagc caatcacatc catcaaactg gagatagtga cctccaaaag tggtggtcag     960
   gcaggggatc agatgtcatg gtcggcaaga gggagcctag cagtgacgat ccatggtggc    1020
   aactatccag ggccctccgg tcccgtcacg ctagtggcct acgaaagagt ggcaacagga    1080
   tccgtcgtta cggtcgctgg ggtgagcaac ttcgagctga tcccaaatcc tgaactagca    1140
   aagaacctgg ttacagaata cggccgattt gacccaggag ccatgaacta cacaaaattg    1200
   atactgagtg agagggaccg tcttggcatc aagaccgtct ggccaacaag ggagtacact    1260
   gactttcgtg aatacttcat ggaggtggcc gacctcaact ctcccctgaa gattgcagga    1320
   gcattcggct tcaaagacat aatccgggcc ataaggagga tagctgtgtg a             1371
<210> 4
   <211> 456
   <212> PRT
   <213> Birnaviridae
<220>
   <221> SOURCE
   <222> 1..456
   <223> /mol_type="protein" /organism="Birnaviridae"
<400> 4
   Met Ser Asn Leu Gln Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Gln Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg Ile Ala Val
       450                 455
<210> 5
   <211> 1371
   <212> DNA
   <213> Birnaviridae
<220>
   <221> source
   <222> 1..1371
   <223> /mol_type="DNA"
   /organism="Birnaviridae" <400> 5
<210> 6
   <211> 456
   <212> PRT
   <213> Birnaviridae
<220>
   <221> SOURCE
   <222> 1..456
   <223> /mol_type="protein" /organi sm="Birnaviridae"
<400> 6
   Met Ser Asn Leu Gln Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Val Gly Gly Glu Leu Val Phe Gln Thr Ser Val His Gly Leu Val
                   245                 250                 255
   Leu Gly Ala Thr Ile Tyr Leu Ile Gly Phe Asp Gly Thr Thr Val Ile
               260                 265                 270
   Thr Arg Ala Val Ala Ala Asn Asn Gly Leu Thr Thr Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Arg Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg Ile Ala Val
       450                 455
<210> 7
   <211> 28
   <212> DNA
   <213> synthetic construct
<220>
   <221> source
   <222> 1..28
   <223> /mol_type="DNA" /note="oligonucleotide for PCR amplification" /organism= synthetic construct"
<400> 7
   ggcgcgccga tgacaaacct gcaagatc 28
<210> 8
   <211> 38
   <212> DNA
   <213> synthetic construct
<220>
   <221> source
   <222> 1..38
   <223> /mol_type="DNA" /note="oligonucleotid für PCR Amplifikation" /organism= synthetic construct"
<400> 8
   ataagaatgc ggccgctcac acagctatcc tccttatg 38
<210> 9
   <211> 35
   <212> DNA
   <213> synthetic construct
<220>
   <221> source
   <222> 1..35
   <223> /mol_type="DNA" /note="oligonucleotid für die PCR-Amplifikation" /organism= "synthetic construct"
<400> 9
   ggcgcgccga tgtctaacct gcaagatcaa accca 35
<210> 10
   <211> 8157
   <212> DNA
   <213> synthetic construct
<220>
   <221> source
   <222> 1..8157
   <223> /mol_type="DNA" /note= "plasmid vector" /organism="synthetic construct"
<400> 10
   aggtggcact tttcggggaa atgtgcgcgg aacccctatt tgtttatttt tctaaataca 60
   ttcaaatatg tatccgctca tgagacaata accctgataa atgcttcaat aatattgaaa 120
   aaggaagagt atgagtattc aacatttccg tgtcgccctt attccctttt ttgcggcatt 180
   ttgccttcct gtttttgctc acccagaaac gctggtgaaa gtaaaagatg ctgaagatca 240
   gttgggtgca cgagtgggtt acatcgaact ggatctcaac agcggtaaga tccttgagag 300
   ttttcgcccc gaagaacgtt ttccaatgat gagcactttt aaagttctgc tatgtggcgc 360
   ggtattatcc cgtattgacg ccgggcaaga gcaactcggt cgccgcatac actattctca 420
   gaatgacttg gttgagtact caccagtcac agaaaagcat cttacggatg gcatgacagt      480
   aagagaatta tgcagtgctg ccataaccat gagtgataac actgcggcca acttacttct      540
   gacaacgatc ggaggaccga aggagctaac cgcttttttg cacaacatgg gggatcatgt      600
   aactcgcctt gatcgttggg aaccggagct gaatgaagcc ataccaaacg acgagcgtga      660
   caccacgatg cctgtagcaa tggcaacaac gttgcgcaaa ctattaactg gcgaactact      720
   tactctagct tcccggcaac aattaataga ctggatggag gcggataaag ttgcaggacc      780
   acttctgcgc tcggcccttc cggctggctg gtttattgct gataaatctg gagccggtga      840
   gcgtgggtct cgcggtatca ttgcagcact ggggccagat ggtaagccct cccgtatcgt      900
   agttatctac acgacgggga gtcaggcaac tatggatgaa cgaaatagac agatcgctga      960
   gataggtgcc tcactgatta agcattggta actgtcagac caagtttact catatatact     1020
   ttagattgat ttaaaacttc atttttaatt taaaaggatc taggtgaaga tcctttttga     1080
   taatctcatg accaaaatcc cttaacgtga gttttcgttc cactgagcgt cagaccccgt     1140
   agaaaagatc aaaggatctt cttgagatcc tttttttctg cgcgtaatct gctgcttgca     1200
   aacaaaaaaa ccaccgctac cagcggtggt ttgtttgccg gatcaagagc taccaactct     1260
   ttttccgaag gtaactggct tcagcagagc gcagatacca aatactgtac ttctagtgta     1320
   gccgtagtta ggccaccact tcaagaactc tgtagcaccg cctacatacc tcgctctgct     1380
   aatcctgtta ccagtggctg ctgccagtgg cgataagtcg tgtcttaccg ggttggactc     1440
   aagacgatag ttaccggata aggcgcagcg gtcgggctga acggggggtt cgtgcacaca     1500
   gcccagcttg gagcgaacga cctacaccga actgagatac ctacagcgtg agctatgaga     1560
   aagcgccacg cttcccgaag ggagaaaggc ggacaggtat ccggtaagcg gcagggtcgg     1620
   aacaggagag cgcacgaggg agcttccagg gggaaacgcc tggtatcttt atagtcctgt     1680
   cgggtttcgc cacctctgac ttgagcgtcg atttttgtga tgctcgtcag gggggcggag     1740
   cctatggaaa aacgccagca acgcggcctt tttacggttc ctggcctttt gctggccttt     1800
   tgctcacatg ttctttcctg cgttatcccc tgattctgtg gataaccgta ttaccgcctt     1860
   tgagtgagct gataccgctc gccgcagccg aacgaccgag cgcagcgagt cagtgagcga     1920
   ggaagcggaa gagcgcccaa tacgcaaacc gcctctcccc gcgcgttggc cgattcatta     1980
   atgcagctgg cacgacaggt ttcccgactg gaaagcgggc agtgagcgca acgcaattaa     2040
   tgtgagttag ctcactcatt aggcacccca ggctttacac tttatgctcc cggctcgtat     2100
   gttgtgtgga attgtgagcg gataacaatt tcacacagga aacagctatg accatgatta     2160
   cgccaagcgc gcaattaacc ctcactaaag ggaacaaaag ctgggtaccg ggcccgcgac     2220
   ctaaccattc aaatgattca taactatctc ctagccagaa ttcgtaccca actcttggga     2280
   aatcaggagg ctgatattcg ccagtaagct tcatagaagt gttcaagttt attttgttag     2340
   caaagatcgt gtacttctga acagtctcaa acccatagta aaatacaact ggggatatac     2400
   gagagttaac cgtgactaca gctagagaac cattagaacc tttttcgaca ctcactccat     2460
   ggatgttttg cttcattaaa tcaatattgt acttcttcca gttcttaaag tccctaggtt    2520
   catcattatt cgttggaggt ctccagaaag tgattgaaga accctcaaac ttgctggaaa    2580
   tttccttacc cttgaccttt aggctttcaa ttttacccaa caatttgtcc aagataaaat    2640
   gcaatccact ggattcaact gagacataac gtttaccgtc gttgatcttc gcagcttttt    2700
   ctgctgtctc tgtaacaaaa tcgggtacct tcaatggaag ttcagcttgg ccccaggcaa    2760
   tttcatgacc tgcctttaga acaccagcat catctttcaa cacggcaaca acataagttg    2820
   tatcagaagg aatagtaaca gattcttctg gctttaaaga tggaacgtcg attgtctttc    2880
   ccgtgtcctt gtcgataaac aataagtggt ctgtcgtaat gaagtcgtgc ttatttgtga    2940
   ttgttacaga tccgtgcgca attttaatat gaacgggttc aataaccttc ttatactcta    3000
   caaggcccgg agtaggatta tgctcactgt tacacaaacc atccatgatg aacactccgt    3060
   catgaacctc ttccttaaag tcaccaccat aagcataagc tttatgcaac ttaccatctg    3120
   cagtactaac atcttcgaat tcaataccgt gatttgccca ttcccagata aagccacctt    3180
   ggtaaaactt ctccttgtag aacaactctt gatattcttt caaagagcca ggaccgttac    3240
   ccattgcatg gccgtactca cacaagatca aaggcttttc aaacttacca ttttcatcag    3300
   tgtggttctt cctccacctt tccataattt caaatgttgg gtacatgaaa ctaaagatat    3360
   ctgcactcaa agcgttcaag tcaccctcat aatgcacaag tctggtagga tccaattgtt    3420
   taattaactt gtacatggct ttgtggtttc tgccataaca agcttcgtta cccaaggacc    3480
   agataataat cgaaggatga ttgacatctc ttaggacaag ttgggaagct ctgtctaagt    3540
   acgcgacctc gtactctgga ttatctgata agtaatgggc attaacatcg tagagtttat    3600
   ttttagtatc tggatattca gcctccaagt tcgtatgacg attaaatggc tcttgaacac    3660
   catgagtttc aagatctgcc tcgtcaatga cccagaagcc cagcttatcg aagaggtcat    3720
   acaccttagg atggtttgga taatgcgagt tacgaacagc attgatgtta aacttcttca    3780
   ttagaatcaa gtccctaaca acaaaatcta atggcacagc tctaccgaac cttggatggt    3840
   gatcatgtct gttgacacct ctaaagagaa tgtctttgcc attaacagta atgttaccgt    3900
   ccttcaactc cacttgtctg aaaccaacat ggtgcttaat agattgaatc acactgccat    3960
   cagatccaat taaatccaac tggtacttgt acaaagtagg attttctgcg gtccaatgtt    4020
   ctggggcctt gacgttgatc ttgaaagctg tttcttcgtt ctttttggtg gagaaggaaa    4080
   taaattcttt agttgaaaaa gtcgtgttcc cattctcctc gttcaacaaa gagcttgcat    4140
   cgtaaacttt agatccatct tcaggttcgt aaagtgtgaa attgatgtga tcataagaag    4200
   aaccctggac atcaactttc acagaaagct ctgcatcctg atactgagag tccacaaaag    4260
   ttgtagtgac cctaacgtct tcaatatggg ccttcttagg caattttagt aaagaaacgt    4320
   ctctgtaaat accagagagc caccattgat cttggtcctc gatataagtg gaatcggacc    4380
   acttgaaaac cttgacgacc actaagtttt cgccctcaga aacgtacttt tggatatcaa    4440
   attcagcccc gttacgggac cccttattga aacccacata ttgaccatta acataaagct    4500
   cgtaacaatt gtccacaccc tcaaatctca atctgtgctc gaacgactca atcgatttcg 4560
   aatctaattc aaaagttcta gcataaacac cagtaggatt tacagtggga ggatttggga 4620
   tgtcgattgg gatagggtac tgtacgttcg tgtaaattgg tttaccgtac ttccagtctt 4680
   cctgaagttc ccaatgggat ggcacagaaa tggtgctcca tttctttgcc gtttcccagt 4740
   ctaaattctt agcatccgga gcgtcaagag gtgcatcaaa caacgcaaaa gcccaaggcc 4800
   cattgagaga ttcgaaaata tcctgatcat agtagtaagc cctagtaggc aatctatttt 4860
   cgtgaacctt tttggggttc cttaaattct caggaataag gcaagccatg gtgccgtcct 4920
   gccgagatat tgtgtacact ggatcaaata ataacacttt caaagtgact aaatcacaat 4980
   tgtcccaaga tatactatag ctctctgttt aacctttata ttgtcaaaaa gggacaatga 5040
   atgaaagtac aaacacaaac acaaacacaa tggaagggag tgtccagggt ggtgattcct 5100
   gactgtactg attcgacgga gttttatttg atttcgttga agtggttaaa gtgaataatt 5160
   cttgaattga gaggaacaaa gagtggataa aataacggaa tggagaggtc cgagcgatga 5220
   ataatgtacg attcggaaga ctatgagccg gctgaacctg aggttatgga ccactaacgt 5280
   cctggttgac aagagtagtc atgtaataca aacgtaaatg tgatatttaa tagaatataa 5340
   gtagatatag ttaaaaagaa gaagaagaat agaaagaata agggtattag aaatttagag 5400
   tcattttaaa caattgataa cttgggttaa agctcgaagt tttgttgata gtagtttttt 5460
   tttttgtttt agttggtttg ttcaatagta taaggttaca gggtgcgaga caaacgttgt 5520
   aacacttttc atctcccccc gctaatcacc tagtcgagag ctcgttttcg acactggatg 5580
   gcggcgttag tatcgaatcg acagcagtat agcgaccagc attcacatac gattgacgca 5640
   tgatattact ttctgcgcac ttaacttcgc atctgggcag atgatgtcga ggcgaaaaaa 5700
   aatataaatc acgctaacat ttgattaaaa tagaacaact acaatataaa aaaactatac 5760
   aaatgacaag ttcttgaaaa caagaatctt tttattgtca gtactgagtc gaggcggccg 5820
   ctggccaccc gggtctagag gcgcgccgtc gacggtacag cttctcgatg agtatgtgtg 5880
   tttatttttt ttttattttt tttgccaaat tctgctcttt cctaaatttc aagtgttgag 5940
   cttgttatcc gctcacaatt ccagcttttg tctcttcacc ttttccaact acaagcgcaa 6000
   cataacaaaa gaataataat tctcctaaga aacacaagcc tcatatacct ttcgagttag 6060
   ggaagaacat cttctctcat gatacacatt gattcgagct attaaatacc tttttctcaa 6120
   tcgaaatctc aagtaaaaca gcaatgaaaa cattacgtaa ctaaaggtgt tcaccactag 6180
   aaatcatacc cttcacactc gacttcaagt agtgaatggt gtagcaacaa agtccaaata 6240
   ccaatgtcaa ccaagtaacc gaccgcacta ctagaaaaag acgctgttgc tcggaccaca 6300
   aatttccgct acacttttca caactatact gaagatacaa aaaacgtgtg tgggtatggc 6360
   tggctaccag gtcgcctggt taaaccaagt caacgtgata catatgtacg ttccaacact 6420
   aagcctaccc taagtttcgg ctcacaggct aggctattat taacatgcaa gacaagggag 6480
   aagcaaagca aagaccaacc gaaacccacc agagcaccct gaactttgcg gtgaacagaa 6540
   ttccgcaaca tatctgagga taccatgatc tcgttttcct actccatatg ggaatcaccc    6600
   actgttgtcc gtaaatatga ccaaattcct accttgattc ctcacgaata atcgcagtcc    6660
   gaaaagccgt tccaaaagcc agtccacagt ccatcaattg gtatgatgat tgtttttttg    6720
   ttcaaactga cacactaacg gtgtggaatg cgaagagtga gcttaccctt ctcctctttg    6780
   ctagcagtac ttgcctacct acctactcta ctacgctgcc atattgtcta acattcggct    6840
   ttctctattt ctacctggcc tggatggctc cgtctcgccc gcctcacaca catacattcc    6900
   tccccctctc gcctgcccca taataattaa acaagttaac aaaaggcgtt acctcttccg    6960
   catcctctcc aatctcatac gattcccctt tcatccgact tacccaacaa gatacaggat    7020
   ctcagtgaaa gatccttcct gccctccctg tctgttgtct actctacatg cgacttggaa    7080
   ggccaaagga ctatcgcatg attattcgcc gggaacccgc gagttccctg ctcttttctt    7140
   tcaaaccagg cagcaaacca ggtgaacaca ctctgatgta gtgcagtccc taagtccttt    7200
   gaagattcgg ggagctagct acccacgcga atgtaacaaa agaacattta cttttgtggg    7260
   gggtggaaaa gtcgattagg atcttgcagc acagaaactg cgcaggggtt tttttcatct    7320
   tggagaagca actggctaaa ttcgacacaa acaaaaactg aaaaatggaa aataaaaaat    7380
   gaaaaagcaa gctgaattcg aagaagggag aattccgcct tctgcaacca cactaatggt    7440
   tggtagtcaa tagatacgca ttagaaggtt actattttat gagtcgatcc ccgcggtgga    7500
   gctccaattc gccctatagt gagtcgtatt acgcgcgctc actggccgtc gttttacaac    7560
   gtcgtgactg ggaaaaccct ggcgttaccc aacttaatcg ccttgcagca catccccctt    7620
   tcgccagctg gcgtaatagc gaagaggccc gcaccgatcg cccttcccaa cagttgcgca    7680
   gcctgaatgg cgaatggcgc gacgcgccct gtagcggcgc attaagcgcg gcgggtgtgg    7740
   tggttacgcg cagcgtgacc gctacacttg ccagcgccct agcgcccgct cctttcgctt    7800
   tcttcccttc ctttctcgcc acgttcgccg gctttccccg tcaagctcta aatcgggggc    7860
   tccctttagg gttccgattt agtgctttac ggcacctcga ccccaaaaaa cttgattagg    7920
   gtgatggttc acgtagtggg ccatcgccct gatagacggt ttttcgccct ttgacgttgg    7980
   agtccacgtt ctttaatagt ggactcttgt tccaaactgg aacaacactc aaccctatct    8040
   cggtctattc ttttgattta taagggattt tgccgatttc ggcctattgg ttaaaaaatg    8100
   agctgattta acaaaaattt aacgcgaatt ttaacaaaat attaacgctt acaattt       8157